# EUROPÄISCHE PATENTSCHRIFT

(11) **EP 2 121 691 B1**
(45) Veröffentlichungstag und Bekanntmachung des Hinweises auf die Patenterteilung: **18.04.2012**
(21) Anmeldenummer: 08715856.4
(22) Anmeldetag: 19.02.2008
(51) Int. Cl.: C07D 471/10, C07D 495/10, C07D 491/107, A61K 31/407, A61P 25/00

(54) **SPIROCYCLISCHE CYCLOHEXAN-DERIVATE**
SPIROCYCLIC CYCLOHEXANE DERIVATIVES
DÉRIVÉS DE CYCLOHEXANE SPIROCYCLIQUES

(30) Priorität: 22.02.2007 DE 102007009319
(43) Veröffentlichungstag der Anmeldung: 25.11.2009
(73) Patentinhaber: Grünenthal GmbH, 52078 Aachen (DE)
(72) Erfinder: SCHUNK, Stefan, 52070 Aachen (DE); SAUNDERS, Derek, 52072 Aachen (DE); HARLFINGER, Stephanie, 50933 Köln (DE); STEUFMEHL, Sonja, 52441 Linnich (DE)
(86) Internationale Anmeldenummer: PCT/EP2008/001271
(87) Internationale Veröffentlichungsnummer: WO 2008/101660

(56) Entgegenhaltungen:
- WO-A-2004/043967
- WO-A-2005/066183
- WO-A-2006/108565

## Beschreibung

Die vorliegende Erfindung betrifft spirocyclische Cyclohexan-Derivate, Verfahren zu deren Herstellung, Arzneimittel enthaltend diese Verbindungen und die Verwendung von spirocyclischen Cyclohexan-Derivaten zur Herstellung von Arzneimitteln.

Das Heptadekapeptid Nociceptin ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist und eine hohe Affinität für den ORL1-Rezeptor aufweist. Der ORL1-Rezeptor ist homolog zu den µ, κ und 5 Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit G_{i/o}-Proteinen zu einer Inhibierung der Adenylatcyclase (Meunier et al., Nature 377, 1995, S. 532-535).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794). Diese Befunde können als Hemmung der stressinduzierten Analgesie erklärt werden (Mogil et al., Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin wirkt antinociceptiv in verschiedenen Schmerzmodellen, beispielsweise im Tail Flick-Test in der Maus (King et al., Neurosci. Lett., 223, 1997, 113-116. In Modellen für neuropathische Schmerzen konnte ebenfalls eine antinociceptive Wirkung von Nociceptin nachgewiesen, die insofern besonders interessant ist, als dass die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf µ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Entsprechend vielfältig sind die Anwendungsmöglichkeiten von Verbindungen, die an den ORL1-Rezeptor binden und diesen aktivieren oder inhibieren. Neben diesem spielen aber gerade im Bereich der Schmerztherapie aber auch anderen der genannten Indikationen Opioidrezeptoren wie der µ-Rezeptor, aber auch die anderen Subtypen dieser Opioidrezeptoren, nämlich δ und κ eine große Rolle. Entsprechend ist es günstig, wenn die Verbindung auch Wirkung an diesen Opioidrezeptoren zeigen.

In WO 2004043967 werden spirocyclische Cyclohexan-Derivate offenbart, die eine hohe Affinität zum ORL1-Rezeptor, aber auch zum µ-Opioid-Rezeptor aufweisen. WO 2004043967 offenbart generisch Verbindungen, bei denen R¹ und R² einen Ring bilden, es werden jedoch keine Beispielverbindungen mit diesem Strukturelement offenbart. Es werden dort ausschließlich Beispielverbindungen offenbart, worin R¹ und R² H oder CH₃ bedeuten, wobei mindestens einer der Reste R¹ und R² H bedeutet. Diese Verbindungen weisen, wie anhand von entsprechenden Daten gezeigt, eine außerordentlich hohe Affinität zum µ-Opioid- bzw. zum ORL1-Rezeptor auf.

Spirocycllsche Cyclohexan-Derivate mit Affinität zum µ-Opioid- bzw. zum ORL1-Rezeptor sind außerdem aus WO 2005066183 bekannt. Es werden dort ebenfalls generische Verbindungen offenbart, bei denen R¹ und R² einen Ring bilden. Es werden Jedoch keine Beispielverbindungen mit diesem Strukturelement offenbart.

Aus WO 2006108565 sind ebenfalls spirocyclische Cyclohexan-Derivate bekannt, die eine hohe Affinität zum µ-Oploid- bzw. zum ORL1-Rezeptor aufweisen. In diesen Verbindungen stehen entweder R¹ und R² für H oder CH₃, wobei R¹ und R² nicht gleichzeitig H bedeuten, oder R¹ und R² bilden zusammen mit -(CH₂)₄- oder -(CH₂)₅- einen Ring.

Metabolische Stabilität ist eine entscheidende Eigenschaft für die Wirksamkeit einer Verbindung und damit auch für den Erfolg einer Arzneimittelentwincklung. Die Verbindungen, die In WO 2004043967 als Beispielverbindungen offenbart sind, werden im Organismus unter anderem über N-Demethylierung abgebaut. Diese Metaboliten sind ihrerseits wieder biologisch aktiv.

In der Arzneimittelentwicklung müssen aktive Metabolite umfassend untersucht werden. Es ist daher vorteilhaft, Verbindungen zu entwickeln, die weniger Metaboliten bilden.

Aufgabe der vorliegenden Erfindung war es, Arzneimittel zur Verfügung zu stellen, die in hohem Maße auf das Nociceptin/ORL1-Rezeptor-System wirken und eine höhere metabolische Stabilität aufweisen als die in WO 2004043967 offenbarten Verbindungen.

Überraschenderweise wurde nun gefunden, dass bestimmte Verbindungen, die zwar generisch in WO 2004043967 beschrieben sind, jedoch nicht anhand von Beispielverbindungen offenbart wurden, eine höhere metabolische Stabilität aufweisen als die offenbarten Beispielverbindungen,

Gegenstand der Erfindung sind daher spirocyclische Cyclohexan-Derivate der allgemeinen Formel I, 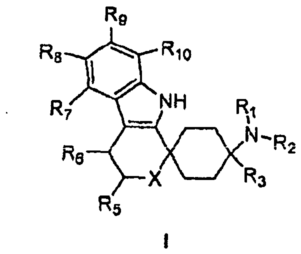 , worin
R¹ und R² gemelnsam einen Ring bilden und für -CH₂CH₂CH₂-stehen
R³ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl oder Heteroaryl, jeweils unsubstituiert oder einfach oder mehrfach substituiert, über C₁₋₃-Alkyl-Gruppe gebundenes Aryl oder C₃₋₈-Cycloalkyl, jeweils unsubstituiert oder einfach oder mehrfach substituiert, steht;
R⁵ für =O; H; C₁₋₅-Alkyl, gesättigt oder ungesättigt, verzweigt oder unverzweigt, unsubstituiert oder einfach oder mehrfach substituiert: COOR¹³, CONR¹³, OR¹³; C₃₋₈-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, steht;
R⁶ für H; F, Cl, NO₂, CF₃, OR¹³, SR¹³, SO₂R¹³, SO₂OR¹³, CN, COOR¹³, NR¹⁴R¹⁵; C₁₋₅-Alkyl, gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, steht;
oder R⁵ und R⁶ gemeinsam (CH₂)ₙ mit n = 2, 3, 4, 5 oder 6 bedeuten, wobei einzelne Wasserstoffatome auch durch F, Cl, Br, I, NO₂, CF₃, OR¹³, CN oder C₁₋₅-Alkyl ersetzt sein können;
R⁷, R⁸, R⁹ und R¹⁰ unabhängig voneinander für H, F, Cl, Br, I, NO₂, CF₃, OR¹³, SR¹³, SO₂R¹³, NHC(=O)NR¹⁴R¹⁵, SO₂NR¹⁴R¹⁵, SO₂OR¹³ CN, COOR¹³, NR¹⁴R¹⁵; C₁₋₅-Alkyl, C₃₋₈-Cycloalkyl, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, stehen;
wobei R¹³ H; C₁₋₅-Alkyl jeweils gesättigt oder ungesättigt, verzweigt oder unverzweigt, unsubstituiert oder einfach oder mehrfach substituiert; C₃₋₈-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, bedeutet;
R¹⁴ und R¹⁵ unabhängig voneinander H; C₁₋₅-Alkyl , jeweils gesättigt oder ungesättigt, verzweigt oder unverzweigt, unsubstituiert oder einfach oder mehrfach substituiert; oder C₃₋₈-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, bedeuten;
oder R¹⁴ und R¹⁵ zusammen CH₂CH₂OCH₂CH₂, CH₂CH₂NR¹⁶CH₂CH₂ oder (CH₂)₃₋₆ bilden,
wobei R¹⁶ H; C₁₋₅-Alkyl gesättigt oder ungesättigt, verzweigt oder unverzweigt, unsubstituiert oder einfach oder mehrfach substituiert, bedeutet;
X für O, S, SO, SO₂ oder NR¹⁷ steht;
R¹⁷ für H; C₁₋₅-Alkyl, gesättigt oder ungesättigt, verzweigt oder unverzweigt; COR¹² oder SO₂R¹²,
wobei R¹² H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert; OR¹³; NR¹⁴R¹⁵ bedeutet;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Bei der Zusammenfassung verschiedener Reste, beispielsweise R⁷, R⁸, R⁹ und R¹⁰ sowie der Zusammenfassung von Resten an deren Substituenten, wie z. B. OR¹³, SR¹³, SO₂R¹³ oder COOR¹³, kann ein Substituent, z.B. R¹³, für zwei oder mehrere Reste, beispielsweise R⁷, R⁸, R⁹ und R¹⁰, innerhalb einer Substanz unterschiedliche Bedeutungen annehmen.

Die erfindungsgemäßen Verbindungen zeigen gute Bindung an den ORL1-Rezeptor und den µ-Opioid-Rezeptor.

Die Ausdrücke "C₁₋₈-Alkyl" "C₁₋₅-Alkyl" und "C₁₋₃-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1, 2, 3, 4, 5, 6, 7 oder 8 C-Atomen bzw. mit 1, 2, 3, 4 oder 5 C-Atomen bzw. 1, 2 oder 3 C-Atomen, d.h. C₁₋₈-Alkanyle, C₂₋₈-Alkenyle und C₂₋₈-Alkinyle bzw. C₁₋₅-Alkanyle, C₂₋₅-Alkenyle und C₂₋₅-Alkinyle bzw. C₁₋₃-Alkanyle, C₂₋₃-Alkenyle und C₂₋₃-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl; Ethylenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), 1,1-Dimethylethyl, 1,1-Dimethylpropyl, Butenyl, Butinyl, Pentenyl, Pentinyl, Hexyl, Hexenyl, Hexinyl, Heptyl, Heptenyl, Heptinyl, Octyl, Octenyl oder Octinyl umfaßt. Besonders bevorzugt im Sinne dieser Erfindung sind Methyl, Ethyl, n-Propyl und n-Butyl.

Der Ausdruck "Cycloalkyl" oder "C₃₋₈-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl enthält. Besonders bevorzugt im Sinne dieser Erfindung sind Cyclobutyl, Cyclopentyl und Cyclohexyl.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen.

Der Ausdruck "Aryl" bedeutet im Sinne dieser Erfindung carbocyclische Ringsysteme mit bis zu 14 Ringgliedern mit mindestens einem aromatischen Ring, aber ohne Heteroatome in nur einem der Ringe, u.a. Phenyle, Naphthyle und Phenanthrenyle, Fluoranthenyle, Fluorenyle, Indanyle und Tetralinyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Besonders vorteilhaft sind Phenyl- oder Naphthyl-Reste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems mit bis zu 14 Ringgliedern sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann.

Im Zusammenhang mit Definitionen von Substituenten bedeutet "Alkyl" "C₁₋₅-Alkyl", soweit "Alkyl" nicht näher spezifiziert ist.

Im Zusammenhang mit "Alkyl" und "Cycloalkyl" versteht man unter dem Begriff "substituiert" im Sinne dieser Erfindung die Substitution eines oder mehrerer Wasserstoffreste durch F, Cl, Br, I, -CN, NH₂, NH-Alkyl, NH-Aryl, NH-Heteroaryl, NH-Cycloalkyl, NH-Alkyl-Aryl, NH-Alkyl-Heteroaryl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)C₁-₆-Alkyl-Aryl, C(=S)C₁₋₆-Alkyl-Aryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, SO-Alkyl, SO₂-Alkyl, SO₂NH₂, SO₃H, PO(O-C₁₋₆-Alkyl)₂ =O, =S, wobei unter mehrfach substituierten Resten solche Reste zu verstehen sind, die entweder an verschiedenen oder an gleichen Atomen mehrfach, z. B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfaßt - OAlkyl u.a. auch -O-CH₂-CH₂-O-CH₂-CH₂-OH. Bevorzugt im Sinne dieser Erfindung ist es, wenn Alkyl oder Cycloalkyl substituiert sind mit F, Cl, Br, I, CN, CH₃, C₂H₅, NH₂, NO₂, SH, CF₃, OH, OCH₃, OC₂H₅ oder N(CH₃)₂. Ganz besonders bevorzugt ist es, wenn Alkyl oder Cycloalkyl substituiert sind mit OH, OCH₃ oder OC₂H₅.

In Bezug auf "Aryl" oder "Heteroaryl" versteht man im Sinne dieser Erfindung unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, NH₂, NH-Alkyl, NH-Aryl, NH-Heteroaryl, NH-Alkyl-Aryl, NH-Alkyl-Heteroaryl, NH-Cycloalkyl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Cycloalkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, C(=S)C₁₋₆-Alkyl-Aryl, C(=O)-Heteroaryl, C(=S)-Heteroaryl, 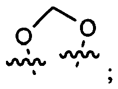 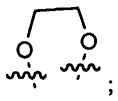 C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, S(O)-Alkyl, S(O)-Aryl, SO₂-Alkyl, SO₂-Aryl, SO₂NH₂, SO₃H, CF₃; Alkyl, Cycloalkyl, Aryl und/oder Heteroaryl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Besonders bevorzugt im Sinne dieser Erfindung ist es, wenn Aryl oder Heteroaryl substituiert sind mit F, Cl, Br, I, CN, CH₃, C₂H₅. NH₂, NO₂, SH, CF₃, OH, OCH₃, OC₂H₅ oder N(CH₃)₂.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier -verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, α-Liponsäure, Acetylglycin, Acetylsalicylsäure, Hippursäure und/oder Asparaginsäure. Besonders bevorzugt ist das Hydrochlorid-Salz, das Citrat und das Hemicitrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt sind das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Bevorzugt sind Verbindungen der allgemeinen Formel I,
wobei "Alkyl substituiert" oder "Cycloalkyl substituiert" Alkyl oder Cycloalkyl substituiert mit F, Cl, Br, I, CN, CH₃, C₂H₅, NH₂, NO₂, SH, CF₃, OH, OCH₃, OC₂H₅ oder N(CH₃)₂ bedeutet und
"Aryl substituiert oder "Heteroaryl substituiert" Aryl oder Heteroaryl substituiert mit F, Cl, Br, I, CN, CH₃, C₂H₅, NH₂, NO₂, SH, CF₃, OH, OCH₃, OC₂H₅ oder N(CH₃)₂ bedeutet,

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen. Bevorzugt sind substituierte Cyclohexanderivate der allgemeinen Formel I, worin
R³ für Phenyl, Benzyl oder Phenethyl, jeweils unsubstituiert oder am Ring ein- oder mehrfach substituiert; C₁₋₅-Alkyl, unsubstituiert oder einfach oder mehrfach substituiert, C₄₋₆-Cycloalkyl, unsubstituiert oder einfach oder mehrfach substituiert; Pyridyl, Thienyl, Thiazolyl, Imidazolyl, 1,2,4 Triazolyl oder Benzlmidazolyl, unsubstituiert oder einfach oder mehrfach substituiert, steht.

Besonders bevorzugt sind spirocyclische Cyclohexanderivate der allgemeinen Formel I, worin R³ Phenyl, Benzyl, Phenethyl, Thienyl, Pyridyl, Thiazolyl, Imidazolyl, 1,2,4 Triazolyl, Benzimidazolyl oder Benzyl, unsubstituiert oder einfach oder mehrfach substituiert mit F, Cl, Br, CN, CH₃, C₂H₅, NH₂, NO₂, SH, CF₃, OH, OCH₃, OC₂H₅ oder N(CH₃)₂; Ethyl, n-Propyl, 2-Propyl, Allyl, n-Butyl, iso-Butyl, sec.-Butyl, tert.-Butyl, n-Pentyl, iso-Pentyl, neo-Pentyl, n-Hexyl Cyclopentyl oder Cyclohexyl steht, jeweils unsubstituiert oder einfach oder mehrfach substituiert mit OH, OCH₃ oder OC₂H₅,
wobei Thienyl, Pyridyl, Thlazolyl, Imidazolyl, 1,2,4 Triazolyl und Benzimidazolyl vorzugsweise unsubstituiert sind;
insbesondere
Phenyl, unsubstituiert oder einfach substituiert mit F, Cl, CN, CH₃; Thienyl; Ethyl, n-Propyl oder n-Butyl, unsubstituiert oder einfach oder mehrfach substituiert mit OCH₃, OH oder OC₂H₅, insbesondere mit OCH₃.
Für eine bevorzugte Ausführungsform der erfindungsgemäßen spirocyclischen Cyclohexan-Derivate gilt, dass
der Rest R⁵ für H, CH₃, COOH, COOCH₃, CH₂OPhenyl, wobei der Phenylrest mit F, Cl, Br, I, CN, CH₃, C₂H₅, NH₂, NO₂, SH, CF₃, OH, OCH₃, OC₂H₅ oder N(CH₃)₂ substituiert sein kann, oder CH₂OH steht.

Besonders bevorzugt sind substituierte Cyclohexan-Derivate, worin R⁵ für H steht.

Bevorzugt sind auch substituierte Cyclohexanderivate der allgemeinen Formel I, worin R⁶ H; Methyl, Ethyl, CF₃, Benzyl oder Phenyl bedeuten kann, wobei der Benzyl- oder Phenylrest mit F, Cl, Br, I, CN, CH₃, C₂H₅, NH₂, NO₂, SH, CF₃, OH, OCH₃, OC₂H₅ oder N(CH₃)₂ substituiert sein kann.

Besonders bevorzugt sind spirocyclische Cyclohexanderivate, worin R⁶ H bedeutet.

Bevorzugt sind weiterhin spirocyclische Cyclohexan-Derivate, worin R⁷ R⁸, R⁹ und R¹⁰ unabhängig voneinander H; C₁₋₅-Alkyl, verzweigt oder unverzweigt, unsubstituiert oder ein- oder mehrfach substituiert; F, Cl, Br, I, CF₃, OH, OCH₃, NH₂, COOH, COOCH₃, NHCH₃ Thienyl, Pyrimidinyl, Pyridyl, N(CH₃)₂ oder NO₂ bedeuten
vorzugsweise
einer der Reste R⁷ R⁸, R⁹ und R¹⁰ für H; C₁₋₅-Alkyl, verzweigt oder unverzweigt, unsubstituiert oder ein- oder mehrfach substituiert; F, Cl, Br, I, OH, OCH₃, COOH, COOCH₃, NH₂, NHCH₃ oder N(CH₃)₂ oder NO₂ steht, während die übrigen Reste H sind,
oder
zwei der Reste R⁷ R⁸, R⁹ und R¹⁰ unabhängig voneinander für H; C₁₋₅-Alkyl, verzweigt oder unverzweigt, unsubstituiert oder ein- oder mehrfach substituiert, F, Cl, Br, I, OH, OCH₃, COOH, COOCH₃, NH₂, NHCH₃ oder N(CH₃)₂ oder NO₂ stehen, während die übrigen Reste H sind.

Besonders bevorzugt sind spirocyclische Cyclohexanderivate, worin R⁷ R⁸, R⁹ und R¹⁰ unabhängig voneinander für H, F, OH, Cl oder OCH₃ stehen.

Verbindungen, bei denen X für O steht, sind besonders bevorzugt. Weiterhin besonders bevorzugt sind Verbindungen der allgemeinen Formel I, X für NR¹⁷ steht.

Ganz besonders bevorzugt sind Verbindungen aus der Gruppe:
4-(Azetidin-1-yl)-4-(3-fluorphenyl)-2',3',4',9'-tetrahydrospiro[cyclohexan-1,1'-pyrido[3,4-b]Indol] (Polareres Diastereomer)
4-(Azetidin-1-yl)-4-(3-fluorphenyl)-2',3',4',9'-tetrahydrospiro[cyclohexan-1,1'-pyrido[3,4-b]indol]( Unpolareres Diastereomer)
4-(Azetidin-1-yl)-4-(3-fluorphenyl)-4',9'-dihydro-3'H-spiro[cyclohexan-1,1'-pyrano[3,4-b]indol](Eines von 2 möglichen Diastereomeren)
1-(4-(Azetidin-1-yl)-4-(3-fluorphenyl)-3',4'-dihydrospiro[cyclohexan-1,1'-pyrido[3,4-b]indol]-2'(9'H)-yl)-3-phenylprop-2-en-1-on (Polareres Diastereomer)
4-(Azetidin-1-yl)-6'-fluor-4-(thiophen-2-yl)-4',9'-dihydro-3'H-spiro[cyclohexan-1,1'-pyrano[3,4-b]indol] 2-hydroxypropan-1,2,3-tricarboxylat (1:1) ( Unpolareres Diastereomer)
   gegebenenfalls auch als Gemisch.

Die erfindungsgemäßen Substanzen wirken beispielsweise auf den im Zusammenhang mit verschiedenen Erkrankungen relevanten ORL1-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes spirocyclisches Cyclohexan-Derivat, sowie gegebenenfalls geeignete Zusatz- und/oder Hilfsstoffe und/oder gegebenenfalls weitere Wirkstoffe.

Die erfindungsgemäßen Verbindungen weisen eine vergleichbare Affinität zum µ-Opioid- bzw. zum ORL1-Rezeptor auf wie die Verbindungen, die als Beispielverbindungen in WO 2004043967 offenbart sind. Im Vergleich zu diesen Verbindungen sind sie jedoch metabolisch stabiler und eignen sich daher besonders für die Arzneimittelentwicklung.

Die erfindungsgemäßen Arzneimittel enthalten neben mindestens einem erfindungsgemäßen spirocyclischen Cyclohexan-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße spirocyclische Cyclohexan-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen spirocyclischen Cyclohexan-Derivate verzögert freisetzen. Die erfindungsgemäßen spirocyclischen Cyclohexan-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens eines erfindungsgemäßen spirocyclischen Cyclohexan-Derivats appliziert.

Für alle vorstehenden Formen der erfindungsgemäßen Arzneimittel ist es besonders bevorzugt, wenn das Arzneimittel neben wenigstens einem spirocyclischen Cyclohexan-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittels liegt ein enthaltenes erfindungsgemäßes spirocyclisches Cyclohexan-Derivat als reines Diastereomer und/oder Enantiomer vor.

Wie in der Einleitung am Stand der Technik abzulesen, wurde der ORL1-Rezeptor insbesondere im Schmerzgeschehen identifiziert. Entsprechend können erfindungsgemäße spirocyclische Cyclohexan-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen spirocyclischen Cyclohexan-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist die Verwendung eines erfindungsgemäßen spirocyclischen Cyclohexan-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes spirocyclisches Cyclohexan-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen spirocyclischen Cyclohexan-Derivats, oder eines erfindungsgemäßen Arzneimittels.

Ein weiterer Gegenstand der Erfindung ist ein Verfahren zur Herstellung der erfindungsgemäßen spirocyclischen Cyclohexan-Derivate wie in der folgenden Beschreibung und Beispielen ausgeführt. Insbesondere geeignet ist dabei ein, Verfahren zur Herstellung eines erfindungsgemäßen spirocyclischen Cyclohexan-Derivats, wobei ein Cyclohexanonderivat der allgemeinen Formel E mit einem Indolderivat der allgemeinen Formel F oder H umgesetzt wird.

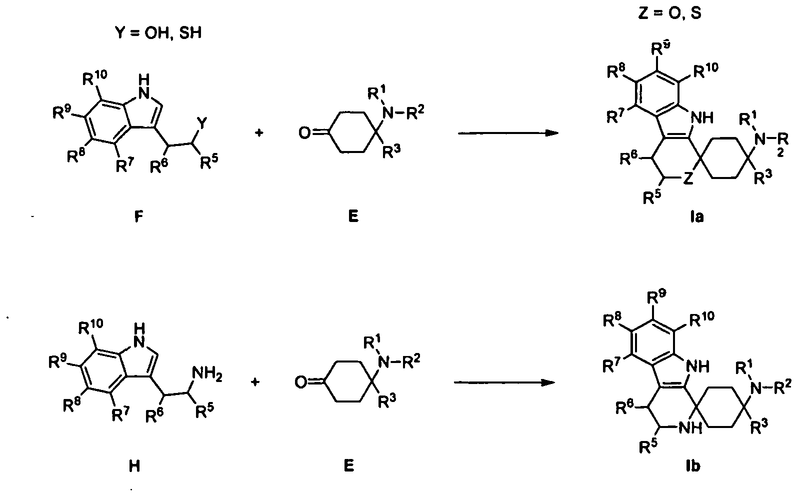

Tryptophole des Typs F (Y = O) können in Reaktionen von Typ der Oxa-Pictet-Spengler-, und Tryptamine des Typs H in Reaktionen von Typ der Pictet-Spengler-Reaktion, mit Ketonen unter Zugabe von mindestens einem geeigneten Reagens aus der Gruppe Säuren, Säureanhydride, Ester oder schwach sauer reagierende Salze oder Lewissäuren unter Bildung von Produkten der Formel **I** zur Reaktion gebracht werden. Für X = SH verläuft die Reaktion analog.

Bevorzugt kommt dabei mindestens ein Reagens aus der Gruppe Carbonsäuren, Phosphorsäuren oder Sulfonsäuren oder deren jeweiligen Anhydride, Carbonsäuretrialkylsilylester, sauer reagierende Salze, Mineralsäuren oder Lewissäuren ausgewählt aus der Gruppe bestehend aus Bortrifluorid, Indium(III)chlorid, Titantetrachlorid, Aluminium(III)chlorid, oder unter Zusatz wenigstens eines Übergangsmetallsalzes, vorzugsweise unter Zusatz wenigstens eines Übergangsmetalltriflats (Übergangsmetalltrifluormethansulfonats), besonders bevorzugt unter Zusatz wenigstens eines Übergangsmetalltrifluormethansulfonats ausgewählt aus der Gruppe bestehend aus Scandium(III)trifluormethansulfonat, Ytterbium(III)trifluormethansulfonat und Indium(III) trifluormethansulfonat, ggf. unter Zusatz von Celite, mit festphasengebundenen Reaktanden oder Reagenzien, bei erhöhter oder erniedrigter Temperatur, mit oder ohne Mikrowelleneinstrahlung, ggf. in einem geeigneten Lösungsmittel oder Lösungsmittelgemisch wie beispielsweise, chlorierten oder unchlorierten, vorzugsweise aromatischen, Kohlenwasserstoffen, Acetonitril; in etherischen Lösungsmitteln, vorzugsweise in Diethylether oder THF; oder in Nitromethan, in geeigneten Fällen auch in Alkoholen oder Wasser, zum Einsatz.

Besonders bevorzugt werden dabei Pyridinium-para-Toluolsulfonat, Phosphorpentoxid in Gegenwart von Celite, Bortrifluorid-etherat, Trifluoressigsäure, Orthotitansäure-tetraisopropylester zusammen mit Trifluoressigsäure, Trifluormethansulfonsäuretrimethylsilylester, Trifluormethansulfonsäure, Methansulfonsäure, Trifluoressigsäure, Essigsäure, Phosphorsäure, Polyphosphorsäure, Polyphosphatester, p-Toluolsulfonsäure, Salzsäure HCl-Gas, Schwefelsäure zusammen mit Acetatpuffer, Zinntetrachlorid, eingesetzt.

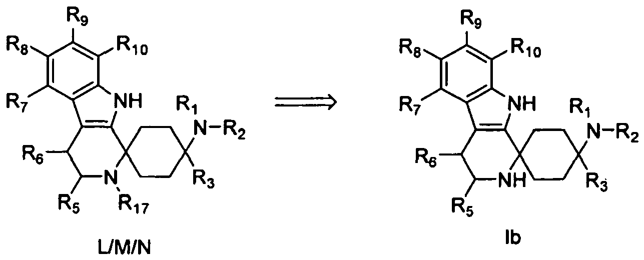

Sekundäre Amine des Typs Ib können nach dem Fachmann bekannten Verfahren zu Verbindungen des Typs **L/M/N** acyliert, sulfonyliert oder carbamoyliert werden. Bevorzugt werden diese Reaktionen bei erhöhter Temperatur, besonders bevorzugt unter Mikrowelleneinstrahlung, durchgeführt.

Eine solche, dem Fachmann bekannte Methode kann die Umsetzung mit einem Anhydrid oder einem Säurechlorid unter Zugabe einer Base, beispielsweise Triethylamin, darstellen.

### Synthese der Ketonbausteine

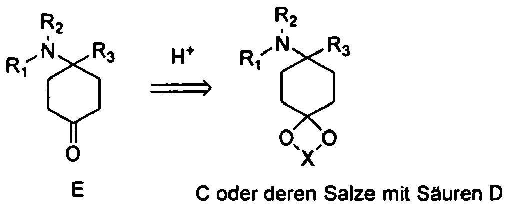

Verbindungen der Formel E können aus entsprechenden Acetalen C, oder aus deren Salzen D, nach dem Fachmann bekannten Methoden durch Entschützen mittels Säuren freigesetzt werden. Dabei ist X aus der Gruppe Alkyl, Alkyl/ Alkyliden/ mit Aryl oder Alkyl (gesättigt/ungesättigt) substituiertem Alkyliden ausgewählt

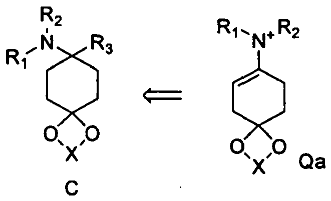

Aminoacetale C mit zwei Substituenten am Stickstoffatom können auch nach dem Fachmann bekannten Verfahren durch Addition von Kohlenstoff-Nucleophilen an Salze von Enaminen Qa erhalten werden, vorzugsweise mit
Organometallverbindungen in inerten Lösungsmitteln.

Die Herstellung von Iminen ist aus der Literatur bekannt.

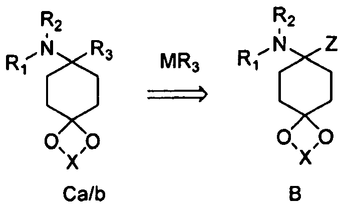

Allgemein können Acetale C auch durch Substitution von geeigneten Abgangsgruppen Z in Strukturen der Formel B erhalten werden. Geeignete

Abgangsgruppen sind bevorzugt Cyanogruppen; 1,2,3-Triazol-1-yl-Gruppen. Weitere geeignete Abgangsgruppen sind 1*H*-Benzo[d][1,2,3]triazol-1-yl-Gruppen und Pyrazol-1-yl-Gruppen (Katritzky et al., Synthesis 1989, 66-69).

Ein besonders bevorzugter Weg zu Verbindungen der Struktur **C** ist die Umsetzung von Aminonitrilen **B** mit entsprechenden Organometallverbindungen, vorzugsweise Grignardverbindungen, vorzugsweise in Ethern, vorzugsweise bei RT. Die Organometallverbindungen sind entweder käuflich erhältlich oder können nach bekannten Methoden hergestellt werden.

Ein weiterer besonders bevorzugter Weg zu Verbindungen der Struktur **C** ist die Umsetzung von Aminotriazolen **B** mit entsprechenden Organometallverbindungen, vorzugsweise Grignardverbindungen, vorzugsweise in Ethern, vorzugsweise bei RT. Die Organometallverbindungen sind entweder käuflich erhältlich oder können nach literaturbekannten Methoden hergestellt werden.

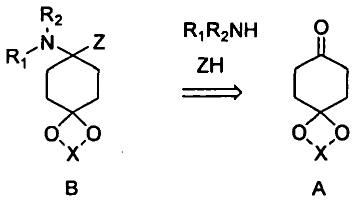

Strukturen der Formel **B** lassen sich durch Reaktion von Ketonen **A** mit Aminen und aciden Reaktanden Z-H herstellen. Geeignete Reaktanden Z-H sind z. B. Cyanwasserstoff, 1,2,3-Triazol, Benzotriazol oder Pyrazol.

Ein besonders bevorzugter Weg zu Verbindungen der Struktur **B** ist die Umsetzung von Ketonen mit Metallcyaniden und dem entsprechenden Amin in Gegenwart von Säure, vorzugsweise in einem Alkohol, bei Temperaturen von - 40 bis 60 °C, vorzugsweise bei Raumtemperatur mit Alkalimetallcyaniden in Methanol.

Ein weiterer besonders bevorzugter Weg zu Verbindungen der Struktur **B** ist die Umsetzung von Ketonen mit 1,2,3-Triazol und dem entsprechenden Amin in Gegenwart unter wasserentziehenden Bedingungen, vorzugsweise unter Verwendung eines Wasserabscheiders bei erhöhter Temperatur in einem inerten Löungsmittel oder unter Verwendung von Molsieb oder einem anderen Trockenmittel. Analog lassen sich **B** analoge Strukturen mit Benzotriazol- oder Pyrazol- statt Triazolgruppen einführen.

Verbindungen der allgemeinen Formeln F und H sind entweder kommerziell erhältlich, oder ihre Herstellung ist aus dem Stand der Technik bekannt oder in für den Fachmann offensichtlicher Weise aus dem Stand der Technik ableitbar. Insbesondere relevant sind hierfür die folgenden Zitate: Jirkovsky et al., J. Heterocycl. Chem., 12, 1975, 937-940; Beck et al., J. Chem. Soc. Perkin 1, 1992, 813-822; Shinada et al., Tetrahedron Lett., 39, 1996, 7099-7102; Garden et al., Tetrahedron, 58, 2002, 8399-8412; Lednicer et al., J. Med. Chem., 23, 1980, 424-430; Bandini et al. J. Org. Chem. 67, 15; 2002, 5386 - 5389; Davis et al., J.Med.Chem. 35, 1, 1992, 177-184; Yamagishi et al., J.Med.Chem. 35, 11, 1992, 2085-2094; Gleave et al.; Bioorg.Med.Chem.Lett. 8, 10, 1998, 1231-1236; Sandmeyer, Helv.Chim.Acta; 2; 1919; 239; Katz et al.; J. Med. Chem. 31, 6, 1988; 1244-1250; Bac et al. Tetrahedron Lett. 1988, 29, 2819; Ma et al. J. Org. Chem. 2001, 66, 4525; Kato et al. J. Fluorine Chem. 99, 1, 1999, 5 - 8.

### Beispiele

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur , "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

**Ketobaustein 1:** 4-Azetidin-1-yl-4-phenylcyclohexanon

1. Stufe: 8-Azetidin-1-yl-1,4-dioxaspiro[4,5]decan-8-carbonitril

Zu einer Mischung von 4 *N* Salzsäure (8,1 ml), Methanol (4,9 ml) und Azetidin (8,5 g, 10 ml, 149 mmol) wurde unter Eiskühlung zuerst 1,4-Dioxaspiro[4,5]decan-8-on (4,84 g, 31 mmol) und danach Kaliumcyanid (4,85 g, 74,4 mmol) in Wasser (15 ml) gegeben. Die Mischung wurde 5 d bei Raumtemperatur gerührt, dann mit Wasser (50 ml) versetzt und mit Diethylether (3 × 50 ml) extrahiert. Die vereinigten organischen Phasen wurden mit Natriumsulfat getrocknet und i. Vak. eingeengt.

*Ausbeute:* 6,77 g (98 %), Öl

*¹H-NMR (DMSO-d₆):* 1,45-1,63 (m, 4H); 1,67-1,82 (m, 4H); 1,99 (q, 2H, J = 7,1 Hz); 3,21 (t, 4H, J = 7,1 Hz); 3,86 (s, 4H).

2. Stufe: 1-(8-Phenyl-1,4-dioxaspiro[4,5]dec-8-yl)azetidin

Eine 2 *M* Lösung von Phenylmagnesiumchlorid in Tetrahydrofuran (12 ml, 24 mmol) wurde unter Argon und Eiskühlung tropfenweise mit einer Lösung von 8-Azetidin-1-yl-1,4-dioxaspiro[4,5]decan-8-carbonitril (2,20 g, 9,9 mmol) in wasserfreiem Tetrahydrofuran (25 ml) versetzt und danach bei Raumtemperatur über Nacht gerührt. Nach Zugabe von gesättigter Ammoniumchloridlösung (5 ml) und Wasser (5 ml) wurden die Phasen getrennt und die wässrige mit Diethylether (3 × 50 ml) extrahiert. Die vereinigten organischen Phasen wurden mit Natriumsulfat getrocknet und i. Vak. eingeengt. Das Rohprodukt wurde durch Flashchromatographie (100 g, 20 × 4,0 cm) mit Ethylacetat / Cyclohexan (1:1) gereinigt.

*Ausbeute:* 670 mg (25 %), farbloses Öl

*¹H-NMR (DMSO-d₆):* 1,27-1,40 (m, 2H); 1,55-2,00 (m, 8H); 2,86 (t, 4H, J = 6,8 Hz); 3,76-3,89 (m, 4H); 7,24-7,45 (m, 5H).

3. Stufe: 4-Azetidin-1-yl-4-phenylcyclohexanon (Ketobaustein 1)

Eine Lösung von 1-(8-Phenyl-1,4-dioxaspiro[4,5]dec-8-yl)azetidin (370 mg, 1,3 mmol) in Aceton (30 ml) wurde mit 6 *N* Salzsäure (2 ml) versetzt und über Nacht bei Raumtemperatur gerührt. Durch Zugabe von 5 *N* Natronlauge wurde pH 10 eingestellt und die wässrige Phase mit Dichlormethan (3 × 20 ml) extrahiert. Die vereinigten organischen Phasen wurden mit Natriumsulfat getrocknet und i. Vak. eingeengt.

*Ausbeute:* 274 mg (92 %), weißer Feststoff

*Schmelzpunkt:* nicht bestimmbar

*¹H-NMR (DMSO-d₆):* 1,67 (td, 2H, J = 13,8, 6,9 Hz); 1,95-2,13 (m, 4H); 2,20-2,33 (m, 2H); 2,40-2,47 (m, 1H); 2,52-2,57 (m, 1H); 2,94 (t, 4H; J = 6,9 Hz); 7,28-7,47 (m, 5H).

**Ketobaustein 2:** 4-Pyrrolidin-4-yl-4-phenylcyclohexanon

1. Stufe: 8-Pyrrolidin-1-yl-1,4-dioxaspiro[4,5]decan-8-carbonitril

Zu einem Gemisch aus 4N Salzsäure (17 ml) und Methanol (10 ml) wurde unter Eiskühlung Pyrrolidin (22,5 ml, 0,306 mol), Cyclohexan-1,4-dion-monoethylenketal (10,0 g, 0,064 mol) und Kaliumcyanid (10,0 g, 0,15 mol) hinzugefügt. Die Mischung wurde 74 h bei Raumtemperatur gerührt und anschließend nach der Zugabe von Wasser (80 ml) mit Diethylether (4 × 70 ml) extrahiert. Nach dem Einengen wurde der Rückstand in Dichlormethan (70 ml) aufgenommen und mit Magnesiumsulfat über Nacht getrocknet. Die organische Phase wurde eingeengt und das Ketal 8-Pyrrolidin-1-yl-1,4-dioxaspiro[4,5]decan-8-carbonitril als weißer Feststoff mit einem Schmelzpunkt von 65-67 °C in einer Ausbeute von 68 % (10,2 g) erhalten.

2. Stufe: 4-(8-Phenyl-1,4-dioxaspiro[4,5]dec-8-yl)pyrrolidin-hydrochlorid

Zu einer 1,82M Phenylmagnesiumchlorid-Lösung in THF (70 ml, 0,127 mol) wurde unter Argon und Eiskühlung innerhalb von 15 min das Aminonitril 8-Pyrrolidin-1-yl-1,4-dioxaspiro[4,5]decan-8-carbonitril (10,0 g, 42,6 mmol), gelöst in THF (90 ml), hinzugefügt und 16 h bei Raumtemperatur gerührt. Zur Aufarbeitung des Reaktionsgemisches wurde unter Eiskühlung gesättigte Ammoniumchloridlösung (100 ml) zugegeben und dann mit Diethylether (3 × 100 ml) extrahiert. Die organische Phase wurde mit Wasser (70 ml) und gesättigter NaCl-Lösung (70 ml) ausgeschüttelt und eingeengt. Es blieb ein gelber Kristallbrei (11,8 g) zurück, der außer der dem gewünschten Produkt noch das Ketal 8-Pyrrolidin-1-yl-1,4-dioxaspiro[4,5]decan-8-carbonitril enthielt. Das Rohprodukt wurde in Ethylmethylketon (70 ml) gelöst und unter Eiskühlung mit CISiMe₃ (8 ml, 0,063 mol) versetzt. Nach einer Reaktionszeit von 6 h konnte das Hydrochlorid 4-(8-Phenyl-1,4-dioxaspiro[4,5]dec-8-yl)pyrrolidin-hydrochlorid in einer Ausbeute von 43 % (5,9 g) als weißer Feststoff isoliert werden.

3. Stufe: 4-Pyrrolidin-4-yl-4-phenylcyclohexanon (Ketobaustein 2)

Das Hydrochlorid 4-(8-Phenyl-1,4-dioxaspiro[4,5]dec-8-yl)pyrrolidin-hydrochlorid (5,8 g, 17,9 mmol) wurde in 7,5N Salzsäure (16 ml) gelöst und 24 h bei Raumtemperatur gerührt. Nach beendeter Hydrolyse wurde die Reaktionsmischung mit Diethylether extrahiert (2 × 50 ml), die wäßrige Phase unter Eiskühlung mit 5N Natronlauge alkalisch gemacht, mit Dichlormethan (3 × 50 ml) extrahiert und eingeengt. Das Keton 4-Pyrrolidin-4-yl-4-phenylcyclohexanon konnte als gelber Feststoff mit einem Schmelzpunkt von 75-79 °C und einer Ausbeute von 96 % (4,1 g) isoliert werden.

**Ketobaustein 3:** 4-Butyl-4-pyrrolidin-1-yl-cyclohexanon

1. Stufe: 1-(8-Pyrrolidin-1-yl-1,4-dioxaspiro[4,5]dec-8-yl)-1*H*-[1,2,3]triazol

Zu einer Lösung von 1,4 Dioxaspiro[4,5]decan-8,on (3,9 g, 25 mmol) in Toluol (40 ml) wurden Pyrrolidin (1,95 g, 2,29 ml, 27,5 mmol), 1,2,3-Triazol (2,07 g, 30 mmol) und Molsieb 4 Å (7,14 g) gegeben. Das Gemisch wurde 7 h bei 90 °C gerührt. Anschließend wurde die Lösung dekantiert und sofort weiter umgesetzt.

2. Stufe: 1-(8-Butyl-1,4-dioxaspiro[4,5]dec-8-yl)pyrrolidin

Zu einer 2 *M* Lösung von n-Butylmagnesiumchlorid (25 ml, 50 mmol) in Tetrahydrofuran wurde unter Eiskühlung und Argon die Reaktionslösung soeben hergestellten Triazol Derivates (ca. 6,9 g, 25 mmol) in Toluol (38 ml) getropft. Das Reaktionsgemisch wurde über Nacht bei Raumtemperatur gerührt und anschließend in gesättigte Ammoniumchloridlösung (60 ml) gegossen. Die Phasen wurden getrennt und die wässrige mit Diethylether (3 × 70 ml) extrahiert. Die vereinigten organischen Phasen wurden mit Natriumsulfat getrocknet, i. Vak. eingeengt und der Rückstand (12 g) durch Flashchromatographie (400 g, 20 x 7,6 cm) mit Ethylacetat / Methanol (9:1) gereinigt.

*Ausbeute:* 2,70 g (40 % über zwei Stufen), braunes Öl

*¹H-NMR (DMSO-d₆):* 0,87 (t, 3H, J = 7,1 Hz); 1,12-1-29 (m, 4H); 1,30-1,45 (m, 4H); 1,46-1,60 (m, 4H); 1,61-1,75 (m, 6H); 1,93 (t, 1H, J = 7,1 Hz); 2,36 (t, 1H, J = 7,0 Hz), 2,58 (br s, 2H), 3,83 (s, 4H).

3. Stufe: 4-Butyl-4-pyrrolidin-1-yl-cyclohexanon (Ketobaustein 3)

Eine Lösung von 1-(8-Butyl-1,4-dioxaspiro[4,5]dec-8-yl)pyrrolidin (2,70 g, 10,1 mmol) in Aceton (100 ml) wurde mit Wasser (10,0 ml) und 37 % Salzsäure (14,0 ml) versetzt und über Nacht bei Raumtemperatur gerührt. Zum Gemisch wurde anschließend langsam 4 *M* Natronlauge getropft, bis pH 10 erreicht war. Das Gemisch wurde mit Diethylether (4 × 40 ml) extrahiert, die vereinigten organischen Phasen wurden mit Natriumsulfat getrocknet und i. Vak. eingeengt. Das Rohprodukt (2,6 g) wurde durch Flashchromatographie (260 g, 30 × 5,6 cm) mit Ethylacetat / Methanol (9:1) gereinigt.

*Ausbeute:* 1,06 g (47 %), braunes Öl

*¹H-NMR (DMSO-d₆):* 0,88 (t, 3H, J = 6,7 Hz); 1,14-1,34 (m, 4H); 1,40-1,50 (m, 2H); 1,62-1,88 (m, 8H); 2,04 (dt, 2H, J = 15,0, 3,9 Hz); 2,42 (ddd, 2H, J = 6,3, 11,8, 15,5 Hz); 2,63 (t, 4H, J = 6,0 Hz).

**Ketobaustein 4:** 4-Benzyl-4-(4-methoxybenzylamino)cyclohexanon

1. Stufe: (1,4-Dioxaspiro[4,5]dec-8-yliden)-(4-methoxybenzyl)amin

Eine Lösung von 1,4-Dioxaspiro[4,5]decan-8-on (4,69 g, 30 mmol) und 4-Methoxybenzylamin (5,35 g, 5,06 ml, 39 mmol) in wasserfreiem Tetrahydrofuran (45 ml) wurde mit Molsieb 4 Å (6 g) versetzt und 20 h bei Raumtemperatur gerührt. Für analytische Zwecke wurde ein aliquoter Teil der Lösung wurde entnommen und i. Vak. eingeengt.

*¹H-NMR (CDC*/*₃):* 1,76-1,87 (m, 2H); 1,91 (t, 2H, J = 6,4 Hz); 2,53 (t, 4H, J = 6,5 Hz); 3,79 (s, 3H); 4,00 (s, 4H); 4,49 (s, 2H); 6,85 (d, 2H, J = 7,9 Hz); 7,21 (d, 2H, J = 8,1 Hz).

Die Probe enthält 4-Methoxybenzylamin.
Das Reaktionsgemisch wurde filtriert, und die Reaktionslösung ohne weitere Aufarbeitung in der nächsten Stufe eingesetzt.

2. Stufe: (8-Benzyl-1,4-dioxaspiro [4,5]dec-8-yl)(4-methoxybenzyl)amin

In einem ausgeheizten Kolben wurde eine 2 *M* Lösung von Benzylmagnesiumchlorid in Tetrahydrofuran (10 ml, 20 mmol) mit einer 0,6 *M* Lösung von (1,4-Dioxaspiro[4,5]dec-8-yliden)-(4-methoxybenzyl)amin in Tetrahydrofuran (17 ml, 10 mmol) unter Argon und Eis-Kühlung langsam tropfenweise versetzt. Das Gemisch wurde 20 h bei Raumtemperatur gerührt und anschließend unter Eis-Wasser-Kühlung zu 20 % Ammoniumchlorid-Lösung (20 ml) getropft. Die organische Phase wurde abgetrennt und die wässrige mit Diethylether (3 × 20 ml) extrahiert. Die vereinigten organischen Phasen wurden mit 2 N Natronlauge (20 ml) und Wasser (20 ml) gewaschen, mit Natriumsulfat getrocknet und i. Vak. eingeengt. Das Rohprodukt (3,7 g) wurde durch Flashchromatographie (370 g, 22 × 7,5 cm) mit Ethylacetat / Cyclohexan (1 : 2) mit 1% Triethylamin gereinigt.

*Ausbeute:* 1,27 g (34 %), gelbliches Öl

*¹H-NMR (CDCl₃):* 1,53-1,66 (m, 6H); 1,89-2,03 (m, 2H); 2,77 (s, 2H); 3,76 (s, 2H); 3,80 (s, 3H); 3,95 (s, 4H); 6,82-6,88 (m, 2H); 7,12-7,37 (m, 7H). Ein austauschbares Proton wurde nicht identifiziert.

3. Stufe: 4-Benzyl-4-(4-methoxybenzylamino)cyclohexanon (Ketobaustein 4)

Zu einer Lösung von (8-Benzyl-1,4-dioxaspiro [4,5 ]dec-8-yl)(4-methoxybenzyl)amin (1,2 g, 3,3 mmol) in Aceton (17 ml) wurde 6 *M* Salzsäure (7 ml) gegeben. Die Reaktionslösung wurde 20 h bei Raumtemperatur gerührt, anschließend mit 25 % Kaliumcarbonat-Lösung basisch (pH-9) gestellt und mit Diethylether (3 × 20 ml) extrahiert. Die vereinigten organischen Phasen wurden mit Natriumsulfat getrocknet und i. Vak. auf ca. 10 ml eingeengt. Der ausgefallene Niederschlag wurde abfiltriert und i. Vak. getrocknet.

*Ausbeute:* 790 mg (74 %), weißer Feststoff

*Schmelzpunkt:* 122-124 °C

¹H-NMR (CDCl₃): 0,96 (br s, 1H); 1,76 (dt, 2H, J = 13,6, 4,6 Hz); 1,84-1,97 (m, 2H); 2,10-2,24 (m, 2H); 2,65-2,80 (m, 2H); 2,86 (s, 2H); 3,81 (s, 3H); 3,87 (s, 2H); 6,85-6,91 (m, 2H); 7,12-7,36 (m, 7H).

**Ketobaustein 5:** 4-(Azetidin-1-yl)-4-(3-fluorphenyl)cyclohexanon

1. Stufe: (1-(8-(3-Fluorphenyl)-1,4-dioxaspiro[4,5]decan-8-yl)azetidin

Eine 1 M Lösung von 3-Fluorphenylmagnesiumbromid in Tetrahydrofuran (250 ml, 250 mmol) wurde unter Argon und Eiskühlung tropfenweise mit einer Lösung von 8-Azetidin-1-yl-1,4-dioxaspiro[4,5]decan-8-carbonitril (13,9 g, 62,53 mmol) in wasserfreiem Tetrahydrofuran (70 ml) versetzt und danach bei Raumtemperatur 24 h gerührt. Anschließend wurde unter Eiskühlung gesättigte Ammoniumchlorid-Lösung (150 ml) zugegeben und 20 min kräftig gerührt. Danach wurden die Phasen getrennt und die wäßrige Phase mit Diethylether (3 x 50 ml) extrahiert. Die vereinigten organischen Phasen wurden mit Natriumsulfat getrocknet und im Vakuum eingeengt. Das Rohprodukt wurde so in einer Ausbeute von 18 g (99 %) als gelbes Öl erhalten.

2. Stufe: ((1-(8-(3-Fluorphenyl)-1,4-dioxaspiro[4,5]decan-8-yl)azetidin Hydrochlorid

Das soeben erhaltene Rohprodukt (18 g, 61,8 mmol) wurde in Ethylmethylketon (100 ml) gelöst, unter Eiskühlung mit ClSiMe₃ (30 ml, 0,237 mol) versetzt und im offenen Kolben bei Raumtemperatur gerührt. Da auch nach 24 h kein Hydrochlorid ausgefallen war, wurde der Ansatz bis zur Trockne eingeengt (20 g braunes Öl, 99 %) und ohne weitere Reinigung für die Ketalspaltung eingesetzt.

3. Stufe: 4-(Azetidin-1-yl)-4-(3-fluorphenyl)cyclohexanon (Ketobaustein 5)

Eine Lösung des soeben erhaltenen Hydrochlorids (20 g, 61 mmol) in Wasser (50 ml) wurde mit konz. Salzsäure (50 ml) und Aceton (50 ml) versetzt und 48 h bei Raumtemperatur gerührt. Durch anschließende Zugabe von 5N Natronlauge wurde pH 11 eingestellt und dann die wäßrige Phase mit Dichlormethan (3 x 100 ml) extrahiert. Die vereinigten organischen Phasen wurden mit Natriumsulfat getrocknet und im Vakuum eingeengt. Das Rohprodukt (15 g) wurde durch Säulenchromatographie [Kieselgel 60 (150 g); Ethylacetat (1000 ml)] gereinigt. Das gewünschte Keton wurde so in einer Ausbeute von 6 g (40 %) erhalten.

**Ketobaustein 6:** 4-(Azetidin-1-yl)-4-(thiophen-2-yl)cyclohexanon

Stufe 1: 1-(8-(Thiophen-2-yl)-1,4-dioxaspiro[4.5]decan-8-yl)azetidin

Eine katalytische Menge Iod wurde zu Magnesium (5,1 g) in 50 ml Diethylether gegeben. Nach 10 Minuten wurde diese Reaktionsmischung mit einer Lösung aus 2-Bromthiophen (5,7 g) in 10 ml THF versetzt. Nach anspringen der Grignard Reaktion wurde 2-Bromthiophen (15 ml) gelöst in 50 ml THF tropfenweise zugegeben und nach Beendigung der Zugabe für zwei Stunden bei Raumtemperatur gerührt.

Zu dieser Reaktionsmischung wurde 8-Azetidin-1-yl-1,4-dioxaspiro[4,5]decan-8-carbonitril (12 g) gelöst in 60 ml THF tropfenweise bei 60-70 °C unter Stickstoffatmosphäre gegeben. Anschließend wurde das Reaktionsgemisch für 1 Stunde bei Raumtemperatur gerührt und der Reaktionsfortschritt dünnschichtchromatograpfisch (50% EtOAc/Hexan) verfolgt.

Nach vollständigem Umsatz wurde das Reaktionsgemisch auf 0°C abgekühlt, mit gesättigter Ammoniumchloridlösung (50 ml) gequencht und abschließend mit Ethylacetat (2x 100 ml) extrahiert. Die vereinigten organischen Phasen wurden über Na₂SO₄ getrocknet. Nach Entfernen des Lösungsmittels unter vermindertem Druck wurde der Rückstand säulenchromatographisch gereinigt (Silica, 5-10% EtOAc/Hexan). Das gewünschte Produkt wurde als brauner Feststoff erhalten (10,2g, 68%).

Stufe 2: 4-(Azetidin-1-yl)-4-(thiophen-2-yl)cyclohexanon (Ketobaustein 6)

Zu einer Lösung von 1-(8-(Thiophen-2-yl)-1,4-dioxaspiro[4.5]decan-8-yl)azetidin (10 g) in 100 ml Methanol wurden bei 0°C 50 ml 6N Salzsäure gegeben und die Reaktionsmischung 1 Stunde bei Raumtemperatur gerührt. Der Reaktionsverlauf wurde mittels Dünnschichtchromatographie (75% EtOAc/Hexan) kontrolliert. Nach vollständigem Umsatz wurde das Methanol abdestilliert, der Rückstand mit Wasser (150 ml) versetzt und mit Ethylacetat (2x 100 ml) extrahiert. Die vereinigten organischen Phasen wurden über Na₂SO₄ getrocknet. Nach Entfernen des Lösungsmittels unter vermindertem Druck wurde der Rückstand in kaltem Wasser (150 ml) aufgenommen, die Lösung für 1 Stunde gerührt und abschließend filtriert. Der zurückbehaltene Feststoff wurde in Ethylacetat aufgenommen und über Na₂SO₄ getrocknet. Nach Entfernen des Lösungsmittels unter vermindertem Druck wurde das gewünschte Produkt (6.5g, 78%) wurden in Form eines braunen Feststoffs erhalten.

### Beispiel A-1

**N-{6'-Fluor-4',9'-dihydro-4-phenyl-spiro[cyclohexan-1,1'(3'H)-pyrano[3,4-b]indol]-4-yl}-azetidin, 2-hydroxy-1,2,3-propantricarboxylat (2:1) (Eines von 2 möglichen Diastereomeren)**

Eine Lösung von 4-Azetidin-1-yl-4-phenylcyclohexanon (Ketobaustein 1) (270 mg, 1,18 mmol) und 5-Fluortryptophol (211 mg, 1,18 mmol) in wasserfreiem Dichlormethan (30 ml) wurde bei 5-10 °C mit Trifluormethansulfonsäure (235 mg, 138 µl, 1,57 mmol) versetzt und bei Raumtemperatur über Nacht gerührt. Nach Zugabe von 0,5 *M* Natronlauge (10 ml) wurden die Phasen getrennt und die wässrige mit Dichlormethan (3 × 10 ml) extrahiert. Die vereinigten organischen Phasen wurden mit Natriumsulfat getrocknet und i. Vak. eingeengt. Das Rohprodukt (280 mg) wurde durch Flashchromatographie (18 g, 20 × 2,0 cm) mit Ethylacetat / Cyclohexan (1:1) und 1 % Triethylamin gereinigt.

*Ausbeute:* 119 mg (29 %), weißer Feststoff
*Schmelzpunkt:* 249-257 °C

*¹H-NMR (DMSO-d₆):* 1,63-1,78 (m, 6H); 2,12 (d, 2H, J = 12,6 Hz); 2,23-2,35 (m, 2H); 2,63 (t, 2H, J = 5,4 Hz); 2,97 (t, 4H, J = 6,7 Hz); 3,85 (t, 2H, J = 5,3 Hz); 6,86 (dt, 1H, J = 9,4, 2,6 Hz); 7,13 (dd, 1H, J = 10,1, 2,5 Hz); 7,26-7,45 (m, 6H); 11,01 (s, 1H).

*¹³C-NMR (DMSO-d₆):* 15,4; 22,0; 46,2; 56,1; 58,7; 71,6; 102,3 (d, J = 23 Hz); 105,3 (d, J = 5 Hz); 108,2 (d, J = 26 Hz); 111,9 (d, J = 10 Hz); 126,4; 126,6; 127,5; 132,4; 140,4; 141,9; 156,7 (d, J = 230 Hz).

Eine Lösung von soeben erhaltenen Spiroethers (119 mg, 0,3 mmol) in heißem Isopropanol (60 ml) wurde mit Citronensäure (72 mg, 0,37 mmol) in Isopropanol (5 ml) versetzt. Der ausgefallene Niederschlag A-1 wurde abfiltriert und getrocknet.

*Ausbeute:* 120 mg (82 %), weißer Feststoff
*Schmelzpunkt:* 189-194 °C

*¹H-NMR (DMSO-d₆):* 1,68-1,83 (m, 6H); 2,13-2,35 (m, 4H), 2,59-2,76 (m, 4H); 3,11 (t, 4H, J = 6,5 Hz); 3,85 (t, 2H, J = 5,3 Hz); 6,87 (dt, 1H, J = 9,5, 2,5 Hz); 7,14 (dd, 1H, J = 9,9, 2,5 Hz); 7,30-7.37 (m, 2H); 7,38-7,48 (m, 4H), 10,95 (s, 1H).

¹³C-NMR (DMSO-d₆). 15,4; 22,1; 26,3 (2 C); 30,3 (2 C); 43,1; 46,7 (2 C); 57,0; 58,8; 71,4; 72,2; 102,4 (d, J = 24 Hz); 105,5 (d, J = 5 Hz); 108,3 (d, J = 26 Hz); 112,0 (d, J = 11 Hz); 126,7 (d, J = 10 Hz); 126,8; 127,7 (2 C); 132,4; 139,7; 141,8; 156,8 (d, J = 230 Hz); 171,4; 175,3,

### Referenzvergleichsbeispiel RV-2

### N-{6'-Fluor-4',9'-dihydro-4-phenyl-spiro[cyclohexan-1,1'(3'H)-pyrano[3,4-b]indol]-4-yl}-pyrrolidin, 2-hydroxy-1,2,3-propantricarboxylat (2:1) (Eines von 2 möglichen Diastereomeren)

Eine Lösung von 4-Pyrrolidin-4-yl-4-phenylcyclohexanon (Ketobaustein 2) (486 mg, 2 mmol) und 5-Fluortryptophol (358 mg, 2 mmol) in wasserfreiem Dichlormethan (20 ml) wurde bei 5-10 °C mit Trifluormethansulfonsäure (399 mg, 232 µl, 2,66 mmol) versetzt und über Nacht bei Raumtemperatur gerührt, Nach Zugabe von 0,5 *M* Natronlauge (10 ml) wurden die Phasen getrennt und die wässrige Phase mit Dichlormethan (3 x 10 ml) extrahiert. Die vereinigten organischen Phasen wurden mit Natriumsulfat getrocknet und 1. Vak. eingeengt. Das Rohprodukt (596 mg) wurde durch Flashchromatographie (18 g, 20 × 1,5 cm) mit Ethylacetat / Cyclohexan (1:9→2:1) und jeweils 1 % Triethylamin gereinigt. Es wurden 2 Fraktionen erhalten.

### Fraktion 1:

*Ausbeute:* 390 mg (48 %), weißer Feststoff
*Schmelzpunkt: >* 260 °C

*¹H-NMR (DMSO-d₆):* 1,60-1,90 (m, 10H); 2,23 (t, 3H, J = 13,1 Hz); 2,39 (d, 3H, J = 12,9 Hz); 2,64 (t, 2H, J = 5,3 Hz); 3,89 (t, 2H, J = 5,3 Hz); 6,88 (dt, 1H. J = 9,4, 2,5 Hz); 7,14 (dd, 1H, J = 9,9, 2,5 Hz); 7,20-7,27 (m, 1H); 7,31-7,40 (m, 5H); 10,85 (s, 1H).

*¹³C-NMR (DMSO-d₆):* 22,1; 23,3; 29,2; 30,5; 44,3; 56,5; 58,7; 71,7; 103,1 (d, J = 23 Hz); 106,2 (d, J = 4 Hz); 109,0 (J = 26 Hz); 112,6 (d, J = 10 Hz); 126,1; 126,3; 126,7; 126,8; 127,0; 127,3; 127,5 (d, J = 10 Hz); 132,4; 140,8; 141,9; 157,5 (d, J = 231 Hz).

### Fraktion 2:

*Ausbeute:* 140 mg (17 %), weißer Feststoff
*Schmelzpunkt:* 188-191 °C

*¹H-NMR (DMSO-d₆):* 1,59 (br s, 4H); 1,76-1,88 (m, 1H); 2,08-2,20 (m, 2H); 2,34-2,48 (m, 3H); 2,52-2,60 (m, 2H); 2,66 (d, 1H, J = 18,5 Hz); 2,80 (t, 3H, J = 7,3 Hz); 3,47 (dd, 2H, J = 13,1, 7,3 Hz); 4,58 (t, 1H, J = 5,3 Hz); 6,22 (s, 1H); 6,77-6,84 (m, 1H); 6,81 (dt, 1H, J = 8,8, 1,9 Hz); 7,12-7,24 (m, 3H); 7,32 (t, 2H, J = 7,6 Hz); 7,48 (d, 2H, J = 7,9 Hz); 10,07 (s, 1H).

*¹³C-NMR (DMSO-d₆):* 22,9; 26,0; 28,6; 28,9; 33,4; 44,8; 58,2; 61,7; 102,7 (d, J = 24 Hz); 107,9 (d, J = 6 Hz); 108,7 (d, J = 26 Hz); 111,4 (d, J = 10 Hz); 125,7; 126,2; 126,8; 127,5; 129,1 (d, J = 10 Hz); 129,3; 131,7; 138,0; 142,2; 156,6 (d, J = 231 Hz).

### Fraktion 1 und Fraktion 2 sind identische Verbindungen.

Eine Lösung der soeben erhaltenen Fraktion 1 (230 mg, 0,57 mmol) in siedenden Isopropanol (180 ml) wurde mit Citronensäure (138 mg, 0,71 mmol) in heißem Isopropanol (10 ml) versetzt. Dabei fiel innerhalb weniger Sekunden ein dicker, weißer Niederschlag A-2 aus, der nach dem Abkühlen abfiltriert wurde.

*Ausbeute:* 150 mg (45 %), weißer Feststoff
*Schmelzpunkt:* 263-270 °C

*¹H-NMR (DMSO-d₆):* 1,65 (br s, 4H); 1,76 (d, 2H; J = 12,5 Hz); 1,88 (t, 2H, J = 13,6 Hz); 2,24 (t, 2H, J = 12,4 Hz); 2,43 (d, 2H, J = 12,9 Hz); 2,52-2,68 (m, 8H); 2,72 (d, 2H, J = 15,3 Hz); 3,88 (t, 2H, J = 5,4 Hz); 6,88 (dt, 1H, J = 9,4, 2,6 Hz); 7,14 (dd, 1H, J = 9,94, 2,47 Hz); 7,22-7,30 (m, 1H); 7,31-7,46 (m, 5H); 10,79 (s, 1H).

*¹³C-NMR (DMSO-d₆):* 22,1; 29,1; 30,4; 43,0; 44,9; 57,6; 58,8; 71,6; 72,2; 102,3; 102,4 (d, J = 23 Hz); 105,6 (d, J = 5 Hz); 108,3 (d, J = 26 Hz); 111,9 (d, J = 11 Hz); 126,6; 126,8; 127,5; 132,4; 140,0; 141,7; 156,7 (d, J = 231 Hz); 171,3; 175,1,

### Referenzvergleichsbeispiel RV-3

### N-{4',9'-Dihydro-4-phenyl-spiro[cyclohexan-1,1'(3'H)-pyrano[3,4-b]indol]-4-yl}-pyrrolidin, 2-hydroxy-1,2,3-propantricarboxylat (4:3) (Unpolareres Diastereomer)

Der Ketobaustein 2 (4-Pyrrolidin-4-yl-4-phenylcyclohexanon) (243 mg, 1 mmol) wurde zusammen mit Tryptophol (161 mg, 1 mmol) in absolutem Dichlormethan (50 ml) vorgelegt. Anschließend erfolgte die Zugabe von Methansulfonsäure (0,13 ml, 2 mmol). Der Ansatz wurde 16 h bei Raumtemperatur gerührt, wobei keine Fällung beobachtet wurde. Die Reaktionsmischung wurde mit 1N NaOH (20 ml) versetzt und eine Stunde gerührt. Die organische Phase wurde abgetrennt und die wäßrige Phase mit Dichlormethan (2 × 20 ml) extrahiert. Die organischen Phasen wurden vereinigt, getrocknet und eingeengt Dabei wurde der gewünschte Spiroether als Diastereoisomerengemisch erhalten (303 mg, 78 %).

Das soeben erhaltene Spiroether Diastereoisomerengemisch (303 mg, 0,78 mmol) wurde 15 min mit Methanol (60 ml) ausgerührt, der Rückstand durch Filtration abgetrennt (248 mg) und aus 2-Propanol (150 ml) umkristallisiert. Es fällt zunächst der unpolarere Spiroether aus (89 mg). Das Filtrat wurde eingeengt und wiederum ein Diastereoisomerengemisch zurückgehalten (103 mg).

Der soeben erhaltene reine unpolarere Spiroether (89 mg, 0,23 mmol) wurde mit Ethanol (45 ml) versetzt und auf 60 °C erwärmt. Zu dieser Suspension wurde Citronensäure in Ethanol (48 mg, 0,25 mmol, 5 ml) gegeben und 10 min bei 60 °C und 1 h bei Raumtemperatur gerührt, Das unpolarere Cltrat A-3 wurde abgesaugt und als farbloser Feststoff (75 mg, 17 %) mit einem Schmelzpunkt von 259 °C isoliert.

### Referenzvergleichsbeispiel RV-4

### N-{4',9'-Dihydro-4-phenyl-spiro[cyclohexan-1,1'(3'H)-pyrano[3,4-b]indol]-4-yl}-pyrrolidin, 2-hydroxy-1,2,3-propantricarboxylat (1:1, Polareres Diastereomer)

Das unter Beispiel A-3 zurückbehaltene Spiroether Diastereoisomerengemisch (103 mg, 0,285 mmol) wurde in Ethanol (80 ml) bei 60 °C gelöst und in der Wärme mit Citronensäure In Ethanol (54 mg, 0,28 mmol, 5 ml) versetzt. Es wurde 1 h bei Raumtemperatur gerührt und das zunächst ausgefallene unpolarere Citrat (85 mg, 19 %) durch Filtration abgetrennt. Das Filtrat wurde auf 2 ml eingeengt, mit Diethylether (40 ml) versetzt, und der ausgefallene farblose Feststoff abgesaugt. Das polarere Citrat A-4 wurde dabei In einer Ausbeute von 16 % (73 mg) und mit einem Schmelzpunkt von 179-180 °C gewonnen.

### Referenzvergleichsbeispiel RV-5

### N-{6'-Fluor-4,9'-dihydro-4-butyl-spiro[cyclohexan-1,1'(3'H)-pyrano[3,4-b]indol]-4-yl}-pyrrolidin, 2-hydroxy-1,2,3-propantricarboxylat (1:1) (Eines von 2 möglichen Diastereomeren)

Eine Lösung von 4-Butyl-4-pyrrolidin-1-yl-cyclohexanon (Ketobaustein 3) (1,06 g, 4,7 mmol) und 2-(5-Fluor-1*H*-3-yl)ethanol (854 mg, 4,7 mmol) in wasserfreiem Dichlormethan (60 ml) wurde unter Eiskühlung und Argon mit Trifluormethansulfonsäure (949 mg, 552 µL, 6,3 mmol) versetzt und ein Tag bei Raumtemperatur gerührt. Danach wurde weitere Trifluormethansulfonsäure (300 mg, 174 µL, 2 mmol) zugesetzt und erneut ein Tag bei Raumtemperatur gerührt. Anschließend wurde das Reaktionsgemisch mit 0,5 *M* Natronlauge (48 ml) versetzt und 20 min gerührt. Die Phasen wurden getrennt, die wässrige Phase mit Dichlormethan (2 × 20 ml) extrahiert und die vereinigten organischen Phasen mit Natriumsulfat getrocknet. Das Rohprodukt (1,8 g) wurde durch Flashchromatographie (180 g, 20 × 5,6 cm) mit Chloroform / Methanol (95:5) gereinigt.

*Ausbeute:* 370 mg (19 %), gelblicher Feststoff (Fraktion 1)
Das Produkt lag als Hydrochlorid vor. Vermutlich stammt der Chlorwasserstoff aus dem zur Chromatographie verwendeten Chloroform.

*¹H-NMR (CDC*/*₃):* 0,97 (t, 3H, J = 6,8 Hz), 1,35-1,41 (m, 4H); 1,46-1,52 (m, 2H); 1,57 (d, 2H, J = 14,6 Hz), 1,89-1,98 (m, 4H); 2,22 (dt, 2H, J = 14,6, 6,0 Hz), 2,35-2,45 (m, 2H); 2,72 (t, 2H, J = 5,3 Hz), 2,78 (dt, 2H, J =14,6, 3,5 Hz); 3,10 (dt, 2H, J = 13,0, 6,9 Hz), 3,63 (dt, 2H, J = 12,2 und 6,6 Hz), 3,92 (t, 2H, J = 5,3 Hz), 6,81 (dt, 1H, J = 9,2 und 2,5 Hz), 7,06 (dd, 1H, J = 9,7, 2,4 Hz), 7,37 (dd, 1H, J = 8,8, 4,5 Hz); 10,36 (br s, 1H); 11,04 (s, 1H).

*¹³C-NMR (CDCl₃):* 13,9; 22,6; 23,4; 25,1; 26,6; 27,0; 29,5; 32,6; 48,2; 60,3; 66,5; 71,0; 102,4 (d, J = 23 Hz); 106,1 (d, J = 4 Hz); 109,2 (d, J = 10 Hz); 112,4 (d, J = 10 Hz); 126,3 (d, J = 10 Hz); 132,4; 139,8; 157,5 (d, J = 233 Hz).

Außerdem wurden noch verunreinigtes Produkt (Fraktion 2, 322 mg, 17 %) und nicht umgesetztes Keton (Fraktion 3, 227 mg, 23 %) erhalten.

Das ¹H-NMR-Spektrum des rohen Produktgemisches zeigte, dass nur ein Diastereoisomer und das Alken entstanden waren, wobei letzteres nicht isoliert wurde.

Eine Lösung von Fraktion 1 (350 mg, 0,83 mmol) in Chloroform (20 ml) wurde mit Natriumhydrogencarbonat-Lösung gewaschen, die organische Phase mit Natriumsulfat getrocknet und i. Vak. eingeengt.

*Ausbeute:* 204 mg (70 %), amorpher, gelblicher Feststoff
*Schmelzpunkt:* 70 °C

*¹H-NMR (CDCl₃):* 0,93 (t, 3H, J = 6,7 Hz), 1,21-1,38 (m, 4H); 1,38-1,42 (m, 2H); 1,48 (d, 2H, J = 12,8 Hz); 1,74 (d, 2H, J = 12,8 Hz); 1,74-1,84 (m, 4H); 1,88 (dt, 2H, J = 13,5, 2,9 Hz); 2,04 (dt, 2H, J = 13,2, 3,2 Hz); 2,69 (t, 4 H, J = 5,8 Hz); 2,74 (t, 2 H, J = 5,4 Hz); 3,99 (t, 2H, J = 5,4 Hz); 6,87 (dt, 1H, J = 9,1, 2,5 Hz); 7,11 (dd, 1H, J = 9,5, 2,4 Hz); 7,23 (dd, 1H, J = 8,7, 4,3 Hz); 7,90 (s, 1H).

*¹³C-NMR (CDCl₃):* 14,2; 22,5; 24,0; 24,1; 24,8; 27,0; 28,6; 30,8; 31,1; 44,1; 54,7; 59,7; 72,4; 103,2 (d, J = 24 Hz); 107,1 (d, J = 5 Hz); 109,4 (d, J = 26 Hz); 111,2 (d, J = 10 Hz); 127,6 (d, J = 10 Hz); 132,0; 141,7; 157,8 (d, J = 234 Hz).

Eine Lösung des soeben erhaltenen gelben Feststoffs (freie Base der Fraktion 1) (180 mg, 0,46 mmol) in heißen Ethanol (15 ml) wurde mit einer heißen Lösung von Citronensäure (90 mg, 0,46 mmol) in Ethanol (1,2 ml) versetzt. Dabei fiel ein weißer Niederschlag aus, der nach dem Abkühlen abfiltriert wurde.

*Ausbeute:* 137 mg (50 %), weißer Feststoff (A-5)*Schmelzpunkt:* 198-199 °C

*¹H-NMR (DMSO-d₆):* 0,92 (t, 3H, J = 6,7 Hz); 1,20-1,40 (m, 4H); 1,44-1,64 (m, 4H); 1,71 (br d, 2H, J = 12,7 Hz); 1,90 (br s, 6H); 2,12 (br t, 2H, J = 12,7 Hz); 2,57 (d, 2H, J = 15,0 Hz); 2,63 (t, 2H, J = 4 Hz); 2,66 (d, 2H, J = 15,0 Hz); 3,07 (br s, 4H); 3,89 (t, 2H, J = 5,1 Hz); 6,87 (dt, 1H, J = 9,1, 2,4 Hz); 7,15 (dd, 1H, J = 9,9, 2,3 Hz); 7,37 (dd, 1H, J = 8,5, 4,4 Hz); 10,64 (s, 1H); ca. 11-12 (sehr br s, 2-3H).

### Referenzvergleichsbeispiel RV-6:

### N-{6'-Fluor-4',9'-dihydro-4-benzyl-spiro[cyclohexan-1,1'(3'H)-pyrano[3,4-b]indol]-4-yl}-amin

**Stufe 1: 4-Benzyl-6'-fluor-N-(4-methoxybenzyl)-4',9'-dihydro-3'H-spiro[cyclohexan-1,1'-pyrano[3,4-b]indol]-4-amin ( Eines von 2 möglichen Diastereomeren)**

Zu einer Lösung von 4-Benzyl-4-(4-methoxybenzylamino)cyclohexanon (Ketobaustein 4) (760 mg, 2,35 mmol) und 2-(5-Fluor-1*H*-indol-3-yl)ethanol (421 mg, 2,35 mmol) In Dichlormethan (50 ml) wurde unter Eis-Wasser-Kühlung Trifluormethansulfonsäure (458 mg, 266µl. 3,05 mmol) getropft. Das Reaktionsgemisch wurde noch 20 h bei Raumtemperatur gerührt. Anschließend wurde die Mischung mit 0,5 *M* Natronlauge (24 ml) versetzt und danach bei Raumtemperatur 2 h gerührt. Die organische Phase wurde abgetrennt und die wässrige mit Dichlormethan (3 × 25 ml) extrahiert. Die vereinigten organischen Phasen wurden mit Natriumchlorid-Lösung (50 ml) gewaschen, mit Natriumsulfat getrocknet und i. Vak. eingeengt.

*Ausbeute:* 1,07 g (94 %), weißer Feststoff
*Schmelzpunkt:* 76-79 °C

*¹H-NMR (CDCl₃):* 1,52 (d, 2H, J = 13 Hz); 1,71-1,95 (m, 4H), 2,07 (dt, 2H, J = 13,1, und 3,3 Hz); 2,74 (t, 2H, J = 5,4 Hz); 2,85 (s, 2H); 3,83 (s, 3H); 3,85 (s, 2H); 3,99 (t, 2H, *J* = 5,34 Hz); 6,81-6,97 (m, 3H); 7,06-7,41 (m, 10H), 7,96 ( br s, 1H). *¹³C-NMR (CDCl₃):* 22,7; 30,7 (4); 44,9; 45,5; 53,6; 54,4; 55,5; 59,9, 72,5, 103,4 (d, J = 24 Hz); 107,4; 109,7 (d, J = 25 Hz); 111,5 (d, J = 10 Hz); 114,2 (2); 126,5; 127,7 (d, J = 10 Hz); 128,4 (2); 129,3 (2); 130,7 (2); 132,3; 133,3; 137,7; 141,5; 158,1 (d, *J* = 233 Hz); 159,1.

Zu einer heißen Lösung des soeben hergestellten Spiroethers (120 mg, 0,25 mmol) in Ethanol (1 ml) wurde eine Lösung von Citronensäure (96 mg, 0,5 mmol) in Ethanol (0,5 ml) gegeben. Nach der Abkühlung wurde die Lösung mit Diethylether (20 ml) versetzt. Der ausgefallene Niederschlag wurde abfiltriert, mit Ethanol und Diethylether gewaschen und i. Vak. getrocknet.

*Ausbeute:* 70 mg (41 %), weißer Feststoff
*Schmelzpunkt:* 135-141 °C

*¹H-NMR (DMSO-d₆):* 1,53-1,84 (m, 6H); 1,95-2,15 (m, 2H); 2,62 (q, 4H, J = 15,3 Hz); 2,55-2,65 (m, 2H); 2,88 (s, 2H); 3,77 (s, 3H); 3,81 (t, 2H, J = 5,3 Hz), 3,97 (s, 2H); 6,86 (dt, 1H, J = 9,3, 2,5 Hz); 6,98 (d, 2H, J = 8,5 Hz); 7,20-7,37 (m, 7H); 7,48 (d, 2H, J = 8,5 Hz); 10,66 (s, 1H).

### Stufe 2: 4-Benzyl-6'-fluor-4',9'-dihydro-3'H-spiro[cyclohexan-1,1'-pyrano(3,4-b]indol]-4-amin (A-6)

Eine Lösung der freien Base des 6'-Fluor-4',9'-dihydro-N-(4-methoxybenzyl)-4-benzyl-spiro[cyclohexan-1,1'(3'H)-pyrano[3,4-b]indol]-4-amin Citrats (200 mg, 0,4 mmol) in Tetrahydrofuran (20 ml) und Methanol (20 ml) wurde mit 10 % Palladium auf Aktivkohle (40 mg) versetzt und 7 h bei 3 bar und 40 °C hydriert. Anschließend wurde das Reaktionsgemisch durch einen Faltenfilter filtriert, der Filterrückstand mit Methanol gewaschen und das Filtrat i. Vak. eingeengt. Das Rohprodukt (186 mg) wurde durch Flashchromatographie (20 g, 21 × 2 cm) mit Methanol gereinigt.

*Ausbeute:* 136 mg (64 %), beigefarbener FeststoffSchmelzpunkt 198-205°C (Zers.) *¹H-NMR (DMSO-d₆):* 1,21-1,38 (m, 2H); 1,52-1,82 (m, 5H); 1,91-2,42 (m, 3H); 2,46-2,71 (m, 4H); 3,82 (t, 2H, J = 5,0 Hz); 6,78-6,89 (m,1H); 7,04-7,31 (m, 7H); 11,0 (s, 0,7H); 11,07 (s, 0,3H).

*¹³C-NMR (DMSO-d₆):* 22,0; 28,0; 28,7; 29,7; 29,9 (2); 31,6; 43,5; 50,0; 50,9; 58,7; 58,8; 71,6; 71,7; 102,4 (d, J = 23 Hz); 105,4 (d, J = 4 Hz); 108,3 (d, J = 26 Hz); 111,7 (d, J = 10 Hz); 126,5 (d, J =10 Hz); 127,7 (2); 130,6 (2C); 132,0; 132,2; 137,3;137,9; 141,9; 156,6 (d, J = 231 Hz).

### Beispiel A-7

### 4-(Azetidin-1-yl)-4-(3-fluorphenyl)-2',3',4',9'-tetrahydrospiro[cyclohexan-1,1'-pyrido[3,4-b]indol] (Polareres Diastereomer)

Der Ketobaustein 5 (742 mg, 3 mmol) und Tryptamin (481 mg, 3 mmol) wurden in MeOH (30 ml) gelöst. Die klare gelbe Reaktionslösung wurde 16 h bei Raumtemperatur gerührt. Anschließend wurde MeOH am Rotationsverdampfer entfernt und der Rückstand in 1,2-Dichlorethan (30 ml) gelöst. Nach Zugabe von Trifluoressigsäure (3 ml, 40 mmol) wurde der Ansatz 3 h bei Raumtemperatur gerührt. Der Verlauf der Reaktion wurde durch DC kontrolliert. Zur Aufarbeitung wurde der Ansatz unter Eiskühlung mit 5N NaOH (50 ml) versetzt. Die wäßrige Phase wurde abgetrennt und mit Dichlormethan (3 × 30 ml) extrahiert. Die vereinigten organischen Phasen wurden über Na₂SO₄ getrocknet und anschließend bis zur Trockne eingeengt. Nach Zugabe von MeOH (20 ml) fiel ein weißer Feststoff aus (630 mg), der ein Gemisch der beiden Diastereoisomeren darstellte. Die beiden Diastereoisomeren konnten durch Säulenchromatographie [Kieselgel 60 (20 g); MeOH (200 ml)] getrennt werden.

Das polarere Produkt (A-7) wurde in einer Ausbeute von 355 mg (30 %) mit einem Schmelzpunkt von 186-188 °C erhalten.

¹³C-NMR (101 MHz; DMSO-d₆) δ ppm: 16,1; 22,8; 25,2; 30,4; 32,1; 34,3; 38,6; 46,0; 51,4; 59,4; 106,6; 110,8; 113,0; 113,2; 114,4; 114,6; 117,2; 117,9; 120,0; 123,7; 126, 9; 129,5; 129,6; 135, 4; 141,3; 141,4; 161, 3; 163, 7.

Das unpolarere Produkt wurde in einer Ausbeute von 110 mg (9 %) mit einem Schmelzpunkt von 277-281 °C erhalten.

¹³C-NMR (101 MHz; DMSO-d₆) δ ppm: 15,4; 22,8; 26,3; 30,4; 31,2; 34,3; 38,5; 46,3; 51,0; 56,5; 106,4; 111,0; 112,9; 113,1; 113,3; 113,5; 117,2; 117,9; 120,0; 122,7; 127,0; 129,0; 129,1; 135,6; 141,9; 144,1; 160, 8; 163,2.

### Beispiel A-8

### 4-(Azetidin-1-yl)-4-(3-fluorphenyl)-2',3',4',9'-tetrahydrospiro[cyclohexan-1,1'-pyrido[3,4-b]indol] (Unpolareres Diastereomer)

Bei Beispiel A-8 handelt es sich um das im Beispiel A-7 erhaltene unpolarere Diastereomer.

### Beispiel A-9

### 4-(Azetidin-1-yl)-4-(3-fluorphenyl)-4',9'-dihydro-3'H-spiro[cyclohexan-1,1'-pyrano[3,4-blindol] (Eines von 2 möglichen Diastereomeren)

Der Ketobaustein 5 (495 mg; 2 mmol) und Tryptophol (322 mg; 2 mmol) wurden in trockenem Dichlormethan (20 ml) gelöst. Bei einer Temperatur von 0 °C wurde Trifluormethansulfonsäure-trimethylsilylester (465 µl, 2,4 mmol) zugegeben. Die rotbraune Suspension wurde 16 h bei Raumtemperatur gerührt. Der Verlauf der Reaktion wurde durch Dünnschichtchromatographie kontrolliert. Zur Aufarbeitung wurde der Ansatz unter Eiskühlung mit 5N NaOH (50 ml) versetzt. Die wäßrige Phase wurde abgetrennt und mit Dichlormethan (3 × 30 ml) extrahiert. Die vereinigten organischen Phasen wurden über Na₂SO₄ getrocknet und anschließend bis zur Trockne eingeengt. Durch Zugabe von MeOH (20 ml) fiel ein weißer Feststoff aus, der eins von zwei möglichen Diastereoisomeren enthielt. Die Mutterlauge enthielt keinen Spiroether mehr.

A-9 wurde so in einer Ausbeute von 240 mg (31 %) mit einem Schmelzpunkt von 270-274 °C erhalten.

1H NMR (400 MHz, DMSO-d₆) δ ppm: 1,64-1,78 (m, 6H), 2,09 (d, *J* = 13,81 Hz, 2H), 2,22-2,40 (m, 2H), 2,66 (t, *J* = 5,40 Hz, 2H), 2,98 (t, *J* = 6,83 Hz, 4H), 3,87 (dd, *J* = 12,39 Hz, 2H), 6,93-6,98 (m, 1H), 7,01-7,09 (m, 1H), 7,12-7,23 (m, 3H), 7,33-7,40 (m, 2 H), 7,42-7,50 (m, 1H), 10,88 (s, 1H)

### Beispiel A-10

**1-(4-(Azetidin-1-yl)-4-(3-fluorphenyl)-3',4'-dihydrospiro[cyclohexan-1,1'-pyrido[3,4-b]indol]-2'(9'H)-yl)-3-phenylprop-2-en-1-on (Polareres Diastereomer)**

Zimtsäurechlorid (371 mg, 2,23 mmol) wurde unter Argon in abs. Tetrahydrofuran (30 ml) gelöst und bei Raumtemperatur mit der freien Base des polareren Spiroamin A-7 (290 mg, 0,744 mmol), gelöst in abs. Tetrahydrofuran (15 ml), innerhalb von 20 min versetzt. Nach einer Reaktionszeit von 1,5 h wurde die trübe Reaktionslösung mit Wasser (10 ml) verdünnt, unter Eiskühlung mit 1 N Natronlauge (10 ml) versetzt und 2 h gerührt. Tetrahydrofuran wurde im Vakuum entfernt. Dabei fiel ein Feststoff aus, der durch Filtration abgetrennt und mit Wasser (3 × 5 ml) gewaschen wurde. Es wurde ein Rohprodukt (350 mg) isoliert, das chromatographisch aufgetrennt wurde [Kieselgel 60 (50 g); Ethylacetat (600 ml)]. Das polarere Amid A-9 wurde so in einer Ausbeute von 192 mg (50 %) mit einem Schmelzpunkt von 120-126 °C erhalten.

*¹³C-NMR (101 MHz; DMSO-D₆) δ ppm:* 22,5; 29,2; 32,6; 37,8; 41,2; 59,4; 60,4; 105,3; 111,0; 113,0; 113,2; 114,4; 114,7; 117,3; 118,3; 120,4; 123,0; 123,7; 126,5; 127, 8; 128, 7; 129,3; 135,1; 135,4; 139, 4; 139,6; 139,7; 140, 4; 161,1; 163, 5; 170,2.

### Beispiel A-11

**4-(Azetidin-1-yl)-6'-fluor-4-(thiophen-2-yl)-4',9'-dihydro-3'H-spiro[cyclohexan-1,1'-pyrano[3,4-b]indol] 2-hydroxypropan-1,2,3-tricarboxylat (1:1) ( Unpolareres Diastereomer)**

Der Ketonbaustein 6 (706 mg, 3 mmol) wurde zusammen mit 5-Fluortryptophol (537 mg, 3 mmol) in Dichlormethan (50 ml) vorgelegt. Anschließend erfolgte unter Eiskühlung die Zugabe von Trifluormethansulfonsäure-trimethylsilylester (0,64 ml, 3,3 mmol), gelöst in Dichlormethan (2 ml). Der Ansatz wurde 24 h bei Raumtemperatur gerührt. Zur Aufarbeitung wurde das Reaktionsgemisch mit Wasser (10 ml) und 2N NaOH (10 ml) versetzt und 20 min bei Raumtemperatur gerührt. Zur weiteren Aufarbeitung des Reaktionsgemisches wurde die organische Phase abgetrennt und die verbliebene wäßrige Phase mit Dichlormethan (3 x 30 ml) ausgeschüttelt. Die vereinigten organischen Extrakte wurden mit Wasser (2 x 20 ml) gewaschen und über Na₂SO₄ getrocknet. Der nach Abdestillieren des Lösungsmittels erhaltene Rückstand (1,2 g) wurde säulenchromatographisch gereinigt [Kieselgel 60 (50 g); Ethylacetat (500 ml)]. Das unpolarere Diastereoisomerwurde in einer Ausbeute von 166 mg (14 %) als hellgelbes Öl erhalten. Das polarere Diastereoisomer wurde in einer Ausbeute von 10 mg (< 1 %) als gelbes Öl erhalten.

Zur Herstellung des Citrates wurde der soeben erhaltene unpolarere Spiroether (160 mg, 0,4 mmol) in heißem Isopropanol (40 ml) gelöst und mit einer ebenfalls heißen, isopropanolischen Citronensäure-Lösung (80 mg, 0,4 mmol in 3 ml) versetzt. Anschließend wurde die Reaktionsmischung 16 h im Kühlschrank gelagert. Der entstandene Feststoff wurde abgesaugt. Das gewünschte Citrat wurde so in einer Ausbeute von 193 mg (78 %) als weißer Feststoff (*Schmelzpunkt:* 174-176 °C) erhalten.

¹³C-NMR (101 MHz, DMSO-d₆) δ ppm: 14,9, 22,0, 28,5, 30,2, 38,9, 42,8, 46,5, 56,8, 58,8, 71,5, 72,3, 102,3, 102,5, 105,5, 108,2, 108,5, 111,9, 112,0, 123,8, 124,3, 126,5, 126,7, 132,4, 141,6, 145,5, 155,6, 157,9, 171,2, 174,8.

### Untersuchungen zur Wirksamkeit der erfindungsgemäßen Verbindungen:

### Messung der ORL1-Bindung

Die Cyclohexan-Derivate der allgemeinen Formel 1 wurden in einem Rezeptorbindungsassay mit ¹³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer Kᵢ-Wert in oder % Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden substituierten spirocyclischen Cyclohexan-Derivates mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem ß-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

In einigen Fällen wurde auf die Bestimmung des Ki-Wertes verzichtet und nur die Hemmung bei einer Testkonzentration von 1 µM bestimmt.

### Vergleichsuntersuchungen

Es wurden jeweils Verbindungen miteinander verglichen, die den gleichen Grundkörper aufwiesen und sich nur in den Resten R¹ und R² unterscheiden. Aufgrund der hohen Affinität der Dimethyl- bzw. Monomethylverbindungen aus WO 2004043967 zum µ-Opioid-Rezeptor und zum ORL1-Rezeptor werden die Affinitäten als Ki-Wert oder als % Hemmung bei einer Testkonzentration von 1 µM angegeben. Diese Testkonzentration ist besonders niedrig und eignet sich zur Detektion von besonders affinen Verbindungen.
1.) R³ = Phenyl, R⁸ = F, R⁵, R⁶, R⁷, R⁹, R¹⁰ = H, X = O

| **Nr.** | **NR¹R²** | **% Hemmung (ORL1) [1 µM]** | **Ki (ORL1) Mittel [µM]** | **% Hemmung (µ) [1µM]** | **KI (µ) Mittel [µM]** |
|---|---|---|---|---|---|
| V-1 | 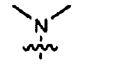 | 99 | 0.0032 | 86 | 0.0027 |
| V-2 | 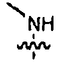 | 91 | 0.0112 | 100 | 0.0008 |
| V-3 | 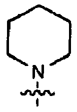 | 0 | | 17 | 0.7367 |
| V-4 | 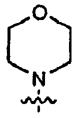 | 2 | | 8 | |
| V-5 | 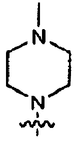 | 76 | | 65 | 1.4100 |
| A-1 | 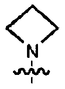 | 91 | 0.0123 | 101 | 0.0019 |
| RV-2 | 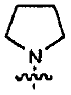 | 56 | 0.3833 | 98 | 0.0018 |
| V-6 | 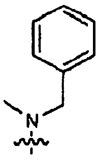 | 18 | | 39 | |
| V-7 | 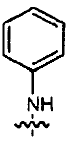 | -8 | 2.9000 | -16 | 6.9433 |

Die beiden Verbindungen V-1 und V-2 weisen eine sehr hohe Affinität zum µ-Opioid, und zum ORL1-Rezeptor auf. Im Falle des µ-Opiold-Rezeptors liegt der Ki-Wert im niedrigen nanomolaren Bereich, im Falle des ORL1-Rezeptors im einstelligen In den niedrigen zweistelligen nanomolaren Bereich. Der Ersatz einer CH₃-Gruppe durch einen Phenyl- oder Benzylrest führt zu Verbindungen, die nur noch eine Affinität im mikromolaren Bereich aufweisen (V-6, V-7). Im Falle des Ringschlussen zwischen den Resten R¹ und R² zum Piperidin-, Morpholin- oder Piperazinring geht die Affinität gleichfalls nicht verloren, sinkt jedoch auf Werte im mikromolaren Bereich. Nur im Fall des Pyrrolidins und des Azetidins bleiben die nanomolaren Kᵢ-Werte für die µ-Oploid-Komponente erhalten. Die Verbindungen besitzen eine höhere metabolische Stabilität im Vergleich zu den Dimethyl-Verbindungen.

Wie in der oben aufgeführten Tabelle gezeigt, besitzt der N-Demethyl-Metabollt von V-1, nämlich V-2, eine ähnlich hohe Aktivität wie die Muttersubstanz V-1. Da ein aktiver Metabollt in der Arznelmitteleniwicklung aufwändigen Untersuchungen unterzogen werden muss, ist die Vermeidung eines Metaboliten von Vorteil. A-1 und RV-2 bilden den N-Demethyl-Metaboliten nicht. Es wurde gezeigt, dass die Umsetzungsraten von A-1 und RV-2 im Vergleich zu V-1 Lebermikrosomen geduzlert ist. Überraschenderweise zeigen A-1 und RV-2 gerade an humanen Lebermikrosomen im Vergleich zu Lebermikrosomen der Maus besonders geringe Umsetzungsraten.
2.) R³ = Phenyl, R⁵, R³, R⁷, R⁸, R⁹, R¹⁰ = H, X = O

| **Nr.** | **NR¹ R²** | **% Hemmung (ORL1) [1µM]** | **Ki (ORL1) Mittel [µM]** | **% Hemmung (µ) [1µM]** | **Ki (µ) Mittel [µM]** |
|---|---|---|---|---|---|
| V-8 | 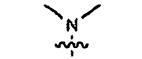 | 98 | 0.0002 | 96 | 0.0012 |
| V-9 | 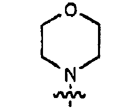 | 5 | | -3 | |
| RV-3 | 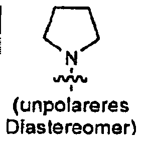 | 95 | 0.0035 | 84 | 0.0011 |
| RV-4 | 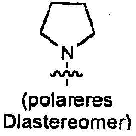 | 61 | 0.11 | 100 | 0.0098 |
| V-10 | 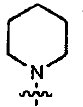 | -2 | | 43 | |
| V-11 | 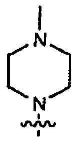 | -12 | | 2 | |

Nur die Verbindung RV-3, worin NR¹R² für Pyrrolidin steht, weist eine mit V-8 vergleichbare Affinität zum µ bzw zum ORL1-Rezeptor auf. Die übrigen Variationen an R¹ und R² führten zu Verschlechterungen der Affinitäten.
3.) R³ = n-Butyl, R⁸ = F, R⁵, R⁶, R⁷, R⁹, R¹⁰ = H, X = O

| **Nr.** | **NR¹ R²** | **% Hemmung (ORL1) [1µM] oder Ki (ORL1) Mittel [µM]** | **% Hemmung (µ) [1µM] oder Ki (µ) Mittel [µM]** |
|---|---|---|---|
| V-12 | 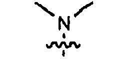 | 0.0016 µM | 0.0009 µM |
| V-13 | 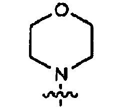 | 6 | 39 |
| RV-5 | 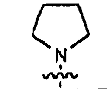 | 47 | 94 |
| V-14 | 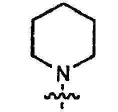 | 9 | 42 |
| V-15 | 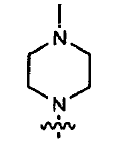 | 8 | 34 |

Auch In der unter 3.) aufgeführten Reihe weist neben der Verbindung V-12 (NR¹R² = Dimethylamin) nur die Verbindung A-5 die eine sehr hohe Affinität zum µ-Opioid-Rezeptor auf.
4.) R³ = Benzyl, R³ = F, R⁵, R⁶, R⁷, R⁹, R¹⁰ = H, X = O

| **Nr.** | **NR¹ R²** | **% Hemmung (ORL1) [1µM]** | **% Hemmung (µ) [1 µM] oder KI (µ) [µM]** |
|---|---|---|---|
| RV-6 | NH₂ | 27 | 90 |
| V-16 1 | 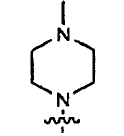 | 11 | 38 |
| V-17 | 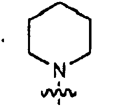 | 5 | 39 |
| V-18 | 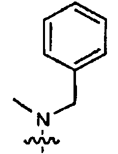 | 10 | 26 |
| V-19 | 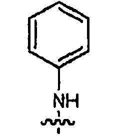 | 8 | -2 |
| V-20 | 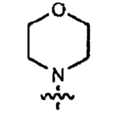 | -3 | 7 |
| V-21 | 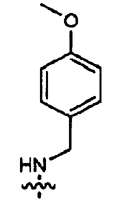 | 49 | 1,2 µM |

In der unter 4.) aufgeführten Vergleichsreihe wird gezeigt dass auch R¹ und R² = H im Vergleich zu verschiedensten anderen Substitutionsmögllchkeiten zu sehr aktiven Verbindungen führen. Auch für diese Verbindungen gilt, dass sie Vorteile bezüglich Metabolismus besitzen.

Für die folgenden Beispiele wurden ebenfalls sehr gute Affinitäten zum µ- bzw. ORL1-Rezeptor festgestellt:

| **Nr.** | **Ki (ORL1) [µM]** | **KI (µ) [µM]** |
|---|---|---|
| A-8 | 0,0008 | 0,0009 |
| A-9 | 0.0048 | 0,0020 |
| A-11 | 0,0066 | 0,0048 |

### Vergleichsuntersuchungen zur metabolische Stabilität

Die metaboische Stabilität der Beispielverbindung A-1 und der Referenzvergleichsbeispielverbindung RV-2 wurde mit der Stabilität der Verbindung V-1 verglichen

Hierfür wurden die Substanzen in-vitro mit Lebermikrosomen (Maus, Mensch) inkubiert und ihre metabolische Stabilität wurde verglichen.

### Methoden:

Für die Mikrosomeninkubationen wurden Stammlösungen von A-1), RV-2 und V-1 zu 10 mmol/l in DMSO angesetzt, diese wurden mit Inkubationspuffer auf 10 µmol/l verdünnt. Dem Inkubationspuffer (100 mmol/l Kaliumphosphat, pH 7.4) waren 4% BSA (Rinderserumalbumin) zugesetzt worden, um die Stabilität der Substanzen in Lösung zu verbessern und unspezifische Verluste durch Adsorptionseffekte zu verhindern. Die Mikrosomen (Maus und Mensch) wurden erst kurz vor dem Versuch aufgetaut und mit Inkubationspuffer auf 3 nmol/ml P450 verdünnt. Die Co-Faktorlösung (10 mmol/l NADP, 10 mmol/l Glukose-6-Phosphat) wurde In inkubationspuffer angesetzt und 5 min bei 37°C vorinkublert.

Die Inkubationsansätze fassten 250 µl: 150 µl Inkubationspuffer + 25 µl 10 µmol/l Substratlösung + 25 µl Mlkrosomenverdünnung (3 nmol P450/mL), gestartet wurde die enzymatische Reaktion durch die Zugabe von 50 µl Co-Faktorlösung. Die Inkubationszeiten betrugen 0,5,10 und 15 min. bei 37°C. Die Reaktionen wurden durch Zugabe von 50 µl Acetonitril abgestoppt.

Neben den zu untersuchenden Substanzen wurde als Positivkontrolle Verepamll mit inkubiert, um die metabolische Aktivität der eingesetzten Mikrosomen sicherzustellen.

Anschließend wurden 50 µl der Inkubationsansätze mit 25 µl Ammoniak alkalisiert und mit 500 µl Methyl-tert.-Butylether extrahiert. Die organische Phase wurde unter Stickstoff eingedampft und in 400 µl 50% Acetonitril/Tetrahydrofuran ():1, v(v), 50% Wasser mit 0.1 % Ameisensäure aufgenommen.

Die Substanzen wurden mittels einer sensitiven und spezifischen LC-MS/MS Methode quantifiziert. Kalibrationsproben (0.1-1 µmol/L) wurden für die einzelnen Analyten in lnkubationspuffer + 4% Rinderserumalbumin angesetzt und mit den Inkubationsproben extrahiert.

### Ergebnis:

Die metabolische Umsatzrate von RV-2 mit Mikrosomen der Maus Ist gegenüber der des V-1 um 22% verringert; mit humanen Mikrosomen geht die Umsatzrate auf rund 30% der des V-1 zurück. Für A-1 war mit humanen Mikrosomen kein Umsatz festzustellen, wobei die Positivkontrolle Verapamil unter denselben Bedingungen adequat umgesetzt wurde. Mit Mikrosomen der Maus geht die Rate von A-1 auf weniger als 10 % der Umsatzrate von V1 zurück.

| Substrat [1 µmol/l] | Umsatzrate humane Mikrosomen (300 pmol/ml) [nmol/min/nmol P450] | Umsatzrate Mikrosomen der Maus (300 pmol/ml) [nmol/min/nmol P450] |
|---|---|---|
| V1 | 0.0300 | 0.0255 |
| RV2 | 0.0105 | 0.0200 |
| A1 | kein Umsatz feststellbar | 0.0009 |

### Schlussfolgerung:

Die Geschwindigkeit der NADP-abhängigen mikrosomalen Biotransfomation wird bei den erfindungsgemäßen Verbindungen gegenüber einer methylierten Aminogruppe verringert. Das Ausmaß dieser Verringerung ist spezlesabhängig und ist mit humanen Mikrosomen stärker zu beobachten als mit Mikrosomen der Maus. Außerdem nimmt auch die Ringstruktur selbst (Vierring/Fünfring) Einfluss auf die Umsatzrate.

### Parenterale Lösung eines spirocyclischen Cyclohexan-Derivats

38 g eines der spirocyclischen Cyclohexan-Derivate, hier Referenzvergleichsbeispiel RV-3 , wird in 1 I Wasser für Injektionszwecke bei Raumtemperatur gelöst und anschließend durch Zugabe von wasserfreier Glukose für Injektionszwecke auf isotone Bedingungen eingestellt.

## Patentansprüche

1. Spirocyclische Cyclohexan-Derivate der allgemeinen Formel I, 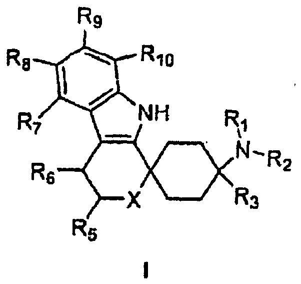 worin
R¹ und R² gemeinsam einen Ring bilden und für -CH₂CHₐCH₂-stehen
R³ für C₁₋₅-Alkyl, jeweils gesättigt oder ungesättigt, verzweigt oder unverzweigt, einfach oder mehrfach substituiert oder unsubstituiert, C₃₋₈-Cycloalkyl, jeweils gesättigt oder ungesättigt, einfach oder mehrfach substituiert oder unsubstituiert; Aryl oder Heteroaryl, jeweils unsubstituiert oder einfach oder mehrfach substituiert; über C₁₋₃-Alkyl-Gruppe gebundenes Aryl oder C₃₋₈-Cycloalkyl, jeweils unsubstituiert oder einfach oder mehrfach substituiert, steht;
R⁵ für =O; H; C₁₋₅-Alkyl, gesättigt oder ungesättigt, verzweigt oder unverzweigt, unsubstituiert oder einfach oder mehrfach substituiert; COOR¹³, CONR¹³, OR¹³; C₃₋₈-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalky) oder Heteroaryl, unsubstituiert oder einfach oder mehrfach substituiert, steht;
R⁶ für H; F, Cl, NO₂, CF₃, OR¹³, SR¹³, SO₂R¹³, SO₂OR¹³, CN, COOR¹³, NR¹⁴R¹⁵; C₁₋₅-Alkyl, gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, steht;
oder R⁵ und R⁶ gemeinsam (CN₂)ₙ mit n = 2, 3, 4, 5 oder 6 bedeuten, wobei einzelne Wasserstoffatome auch durch F, Cl, Br, I, NO₂, CF₃, OR¹³, CN oder C₁₋₅-Alkyl ersetzt sein können;
R⁷, R⁸, R⁹ und R¹⁰ unabhängig voneinander für
H, F, Cl, Br, I, NO₂, CF₃, OR¹³, SR¹³, SO₂R¹³, SO₂OR¹³, NHC(=O)NR¹⁴R¹⁵, SO₂NR¹⁴R¹⁵, CN, COOR¹³, NR¹⁴R¹⁵, C₁₋₅-Alkyl, C₃₋₈-Cycloalkyl, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, stehen;
wobei R¹³ H; C₁₋₅-Alkyl jeweils gesättigt oder ungesättigt, verzweigt oder unverzweigt, unsubstituiert oder einfach oder mehrfach substituiert; C₃₋₈-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl,
unsubsütuiert oder einfach oder mehrfach substituiert, bedeutet;
R¹⁴ und R¹⁵ unabhängig voneinander H; C₁₋₅-Alkyl , jeweils gesättigt oder ungesättigt, verzweigt oder unverzweigt, unsubstituiert oder einfach oder mehrfach substituiert; oder C₃₋₈-Cycloalkyl, jeweils gesättigt oder ungesättigt, unsubstituiert oder einfach oder mehrfach substituiert; Aryl-, oder Heteroaryl, unsubstituiert oder einfach oder mehrfach substituiert; oder über C₁₋₃-Alkyl gebundenes Aryl, C₃₋₈-Cycloalkyl oder Heteroaryl, unsubstituiert oder einfach oder mehrfach substituiert, bedeuten;
oder R¹⁴ und R¹⁵ zusammen CH₂CH₂OCH₂CH₂, CH₂CH₂NR¹⁶CH₂CH₂ oder (CH₂)₃₋₈ bilden,
wobei R¹⁶ H; C₁₋₅₋Alkyl gesättigt oder ungesättigt, verzweigt oder unverzweigt, unsubstituiert oder einfach oder mehrfach substituiert, bedeutet;
X für O, S, SO, SO₂ oder NR¹⁷ steht;
R¹⁷ für H; C₁₋₅-Alkyl, gesättigt oder ungesättigt, verzweigt oder unverzweigt; COR¹² oder SO₂R¹²,
wobei R¹² H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert; OR¹³; NR¹⁴R¹⁵ bedeutet;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

2. Spirocyclische Cyclohexanderivate gemäß Anspruch 1,
wobei "Alkyl substituiert" oder "Cycloalkyl substituiert" Alkyl oder Cycloalkyl substituiert mit F, Cl, Br, I, CN, CH₃, C₂H₅, NH₂, NO₂, SH, CF₃, OH, OCH₃, OC₂H₅ oder N(CH₃)₂ bedeutet und
"Aryl substituiert oder "Heteroaryl substituiert" Aryl oder Heteroaryl substituiert mit F, Cl, Br, I, CN, CH₃, C₂H₅, NH₂, NO₂, SH, CF₃, OH, OCH₃, OC₂H₅ oder N(CH₃)₂ bedeutet.

3. Spirocyclische Cyclohexanderivate gemäß Anspruch 1, worin
R³ für Phenyl, Benzyl oder Phenethyl, jeweils unsubstituiert oder am Ring ein- oder mehrfach substituiert; C₁₋₅-Alkyl, unsubstituiert oder einfach oder mehrfach substituiert; C₄₋₈-Cycloalkyl, unsubstituiert oder einfach oder mehrfach substituiert; Pyridyl, Thienyl, Thiazolyl, Imidazolyl, 1,2,4 Triazolyl oder Benzimidazolyl, unsubstituiert oder einfach oder mehrfach substituiert, steht.

4. Spirocyclische Cyclohexanderivate gemäß Anspruch 3, worin R³ für Phenyl, unsubstituiert oder einfach substituiert mit F, Cl, CN, CH₃; Thienyl; Ethyl, n-Propyl oder n-Butyl, unsubstituiert oder einfach oder mehrfach substituiert mit OCH₃, OH oder OC₂H₅, insbesondere mit OCH₃, steht.

5. Spirocyclische Cyclohexan-Derivate gemäß Anspruch 1, worin
der Rest R⁵ für H, CH₃, COOH, COOCH₃, CH₂OPhenyl, wobei der Phenylrest mit F, Cl, Br, I, CN, CH₃, C₂H₅, NH₂, NO₂, SH, CF₃, OH, OCH₃, OC₂H₅ oder N(CH₃)₂ substituiert sein kann, oder CH₂OH steht.

6. Spirocyclische Cyclohexan-Derivate gemäß Anspruch 1, worin
R⁶ H; Methyl, Ethyl, CF₃, Benzyl oder Phenyl bedeuten kann, wobei der Benzyl- oder Phenylrest mit F, Cl, Br, I, CN, CH₃, C₂H₅, NH₂, NO₂, SH, CF₃, OH, OCH₃, OC₂H₅ oder N(CH₃)₂ substituiert sein kann.

7. Spirocyclische Cyclohexan-Derivate gemäß Anspruch 1, worin
R⁷ R⁸, R⁹ und R¹⁰ unabhängig voneinander H; C₁₋₅-Alkyl, verzweigt oder unverzweigt, unsubstituiert oder ein- oder mehrfach substituiert; F, Cl. Br. I, CF₃, OH. OCH₃, NH₂, COOH, COOCH₃, NHCH₃ Thienyl, Pyrimidinyl. Pyridyl, N(CH₃)₂ oder NO₂ bedeuten.

8. Spirocyclische Cyclohexan-Derivate gemäß einem der Ansprüche 1 bis 7, worin X für O steht.

9. Spirocyclische Cyclohexan-Derivate gemäß einem der Ansprüche 1 bis 7, worin X für NR¹⁷ steht.

10. Spirocyclische Cyclohexan-Derivate gemäß Anspruch 1 aus der Gruppe
N-{6'-Fluor-4',9'-dihydro-4-phenyl-spiro-[cyclohexan-1,1'(3'H)-pyrano[3,4-b]indol]-4-yl}-azetidin, 2-hydroxy-1,2,3-propantricarboxylat (2:1) 4-(Azetidin-1-yl)-4-(3-fluorphenyl)-2',3',4',9'-tetrahydrospiro[cyclohexan-1,1'-pyrido[3,4-b1]indol] (Polareres Diastereomer)
4-(Azetidin-1-yl)-4-(3-fluorphenyl)-2',3',4',9'-tetrahydrospiro[cyclohexan-1,1'-pyrido[3,4-b]indol] (Unpolareres Diastereomer)
4-(Azetidin-1-yl)-4-(3-fluorphenyl)-4',9'-dihydro-3'H-spiro[cyclohexan-1,1'-pyrano[3,4-b]indol] (Eines von 2 möglichen Diastereomeren)
1-(4-(Azetidin-1-yl)-4-(3-fluorphenyl)-3',4'-dihydrospiro[cyctohexan-1,1'-pyrido[3,4-b]indol]-2'(9'H)-yl)-3-phenylprop-2-en-1-one (Polareres Diastereomer) 4-(Azetidin-1-yl)-6'-fluor-4-(thiophen-2-yl)-4',9'-dihydro-3'H-spiro[cyclohexan-1,1'-pyrano[3,4-b]indol] 2-hydroxypropan-1,2,3-tricarboxylat (1:1) ( Unpolareres Diastereomer)
gegebenenfalls auch als Gemisch.

11. Verfahren zur Herstellung von spirocyclischen Cyclohexanderivaten gemäß Anspruch 1, **dadurch gekennzeichnet, dass** ein Edukt der allgemeinen Formel E 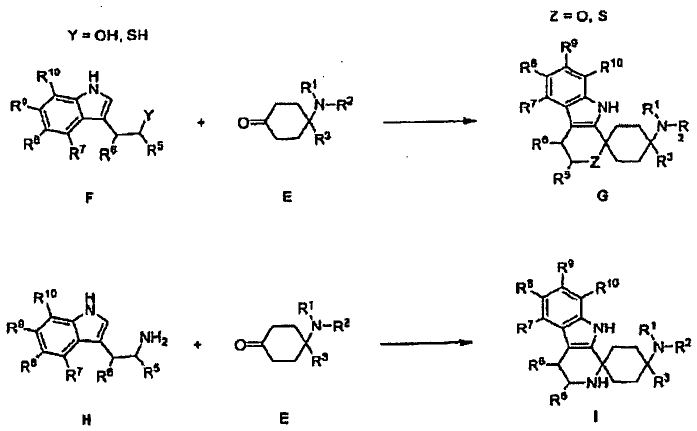 unter Zugabe von Säure, oder deren Trimethylsilylester, beispielsweise Trifluormethansulfonsäuretrimethylsilylester, Trifluormethansulfonsäure. Essigsäure, Phosphorsäure, Methansulfonsäure oder Trifluoressigsäure, in einem geeigneten Lösungsmittel, beispielsweise Dichlorethan, Dichlormethan, Chloroform, Acetonitril, Diethylether oder Nitromethan, mit einem Edukt der allgemeinen Formel F oder H umgesetzt wird, wobei die Reste R¹ bis R³ und
R⁵ bis R¹⁰ die in Anspruch 1 angegebenen Bedeutungen haben.

12. Verfahren zur Herstellung von spirocyclischen Cyclohexanderivaten gemäß Anspruch 1, bei denen X NR¹⁷ und R¹⁷ COR¹² oder SO₂R¹² bedeutet,
**dadurch gekennzeichnet, dass** ein spirocyclisches Cyclohexanderivat, bei dem X NH bedeutet, unter Zugabe von Base, beispielsweise Triethylamin, mit einem Anhydrid oder einem Säurechlorid umgesetzt wird, vorzugsweise unter Mikrowelleneinstrahlung.

13. Verfahren zur Herstellung von spirocyclischen Cyclohexanderivaten gemäß Anspruch 1, bei denen X SO oder SO₂ bedeutet, **dadurch gekennzeichnet,**
**dass** ein spirocyclisches Cyclohexanderivat, bei dem X S bedeutet, mit Hilfe eines Oxidationsmittels, beispielsweise H₂O₂, oxidiert wird.

14. Arzneimittel enthaltend wenigstens ein spirocyclisches Cyclohexan-Derivat gemäß einem der Ansprüche 1 bis 10, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatzund/oder Hilfsstoffe und/oder gegebenenfalls weiterer Wirkstoffe.

15. Verwendung eines spirocyclischen Cyclohexan-Derivates gemäß einem der Ansprüche 1 bis 10, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem, neuropathischem oder chronischem Schmerz.

16. Verwendung eines spirocyclisches Cyclohexan-Derivats gemäß einem der Ansprüche 1 bis 10 zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmissbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

1. Spirocyclic cyclohexane derivatives having the general formula I, 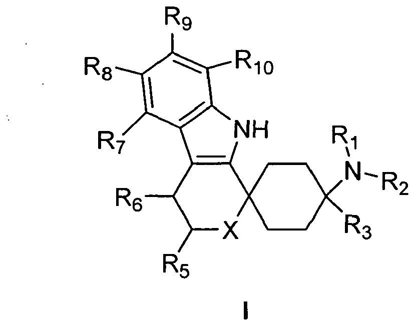 wherein
R¹ and R² together form a ring and denote -CH₂CH₂CH₂-R³ denotes C₁₋₅ alkyl, in each case saturated or unsaturated, branched or unbranched, mono- or polysubstituted or unsubstituted; C₃₋₈ cycloalkyl, in each case saturated or unsaturated, mono- or polysubstituted or unsubstituted; aryl or heteroaryl, in each case unsubstituted or mono- or polysubstituted; aryl or C₃₋₈ cycloalkyl bonded via a C₁₋₃ alkyl group, in each case unsubstituted or mono- or polysubstituted;
R⁵ denotes =O; H; C₁₋₅ alkyl, saturated or unsaturated, branched or unbranched, unsubstituted or mono- or polysubstituted; COOR¹³, CONR¹³, OR¹³; C₃₋₈ cycloalkyl, saturated or unsaturated, unsubstituted or mono- or polysubstituted; aryl or heteroaryl, unsubstituted or mono- or polysubstituted; or aryl, C₃₋₈ cycloalkyl or heteroaryl bonded via C₁₋₃ alkyl, unsubstituted or mono- or polysubstituted;
R⁶ denotes H; F, Cl, NO₂, CF₃, OR¹³, SR¹³, SO₂R¹³, SO₂OR¹³, CN, COOR¹³, NR¹⁴R¹⁵; C₁₋₅ alkyl, saturated or unsaturated, branched or unbranched, unsubstituted or mono-or polysubstituted; C₃₋₈ cycloalkyl, saturated or unsaturated, unsubstituted or mono- or polysubstituted; aryl or heteroaryl, unsubstituted or mono- or polysubstituted; or aryl, C₃₋₈ cycloalkyl or heteroaryl bonded via C₁₋₃ alkyl, unsubstituted or mono- or polysubstituted;
or R⁵ and R⁶ together denote (CH₂)ₙ where n = 2, 3, 4, 5 or 6, wherein individual hydrogen atoms can also be replaced by F, Cl, Br, I, NO₂, CF₃, OR¹³, CN or C₁₋₅ alkyl;
R⁷, R⁸, R⁹ and R¹⁰ independently of one another denote
H, F, Cl, Br, I, NO₂, CF₃, OR¹³, SR¹³, SO₂R¹³, SO₂OR¹³, NHC(=O)NR¹⁴R¹⁵, SO₂NR¹⁴R¹⁵, CN, COOR¹³, NR¹⁴R¹⁵; C₁₋₅ alkyl, C₃₋₈ cycloalkyl, unsubstituted or mono-or polysubstituted; aryl or heteroaryl, unsubstituted or mono- or polysubstituted; or aryl, C₃₋₈ cycloalkyl or heteroaryl bonded via C₁₋₃ alkyl, unsubstituted or mono- or polysubstituted;
wherein R¹³ denotes H; C₁₋₅ alkyl, in each case saturated or unsaturated, branched or unbranched, unsubstituted or mono- or polysubstituted; C₃₋₈ cycloalkyl, in each case saturated or unsaturated, unsubstituted or mono- or polysubstituted; aryl or heteroaryl, unsubstituted or mono- or polysubstituted; or aryl, C₃₋₈ cycloalkyl or heteroaryl bonded via C₁₋₃ alkyl, unsubstituted or mono- or polysubstituted;
R¹⁴ and R¹⁵ independently of each other denote H; C₁₋₅ alkyl, in each case saturated or unsaturated, branched or unbranched, unsubstituted or mono- or polysubstituted; or C₃₋₈ cycloalkyl, in each case saturated or unsaturated, unsubstituted or mono- or polysubstituted; aryl or heteroaryl, unsubstituted or mono- or polysubstituted; or aryl, C₃₋₈ cycloalkyl or heteroaryl bonded via C₁₋₃ alkyl, unsubstituted or mono- or polysubstituted;
or R¹⁴ and R¹⁵ together form CH₂CH₂OCH₂CH₂, CH₂CH₂NR¹⁶CH₂CH₂ or (CH₂)₃₋₆, wherein R¹⁶ denotes H; C₁₋₅ alkyl, saturated or unsaturated, branched or unbranched, unsubstituted or mono- or polysubstituted;
X denotes O, S, SO, SO₂ or NR¹⁷;
R¹⁷ denotes H; C₁₋₅ alkyl, saturated or unsaturated, branched or unbranched; COR¹² or SO₂R¹²,
wherein R¹² denotes H; C₁₋₅ alkyl, in each case saturated or unsaturated, branched or unbranched, mono- or polysubstituted or unsubstituted; C₃₋₈ cycloalkyl, in each case saturated or unsaturated, mono- or polysubstituted or unsubstituted; aryl or heteroaryl, in each case mono- or polysubstituted or unsubstituted; or aryl, C₃₋₈ cycloalkyl or heteroaryl bonded via C₁₋₃ alkyl, in each case mono- or polysubstituted or unsubstituted; OR¹³, NR¹⁴R¹⁵;
in the form of the racemate; the enantiomers, diastereomers, mixtures of enantiomers or diastereomers or a single enantiomer or diastereomer; the bases and/or salts of physiologically compatible acids or cations.

2. Spirocyclic cyclohexane derivatives according to claim 1,
wherein "alkyl substituted" or "cycloalkyl substituted" denotes alkyl or cycloalkyl substituted with F, Cl, Br, I, CN, CH₃, C₂H₅, NH₂, NO₂, SH, CF₃, OH, OCH₃, OC₂H₅ or N(CH₃)₂ and
"aryl substituted" or "heteroaryl substituted" denotes aryl or heteroaryl substituted with F, Cl, Br, I, CN, CH₃, C₂H₅, NH₂, NO₂, SH, CF₃, OH, OCH₃, OC₂H₅ or N(CH₃)₂.

3. Spirocyclic cyclohexane derivatives according to claim 1, wherein R³ denotes phenyl, benzyl or phenethyl, each unsubstituted or mono- or polysubstituted at the ring; C₁₋₅ alkyl, unsubstituted or mono- or polysubstituted; C₄₋₆ cycloalkyl, unsubstituted or mono- or polysubstituted; pyridyl, thienyl, thiazolyl, imidazolyl, 1,2,4-triazolyl or benzimidazolyl, unsubstituted or mono- or polysubstituted.

4. Spirocyclic cyclohexane derivatives according to claim 3, wherein R³ denotes phenyl, unsubstituted or monosubstituted with F, Cl, CN, CH₃; thienyl; ethyl, n-propyl or n-butyl, unsubstituted or mono- or polysubstituted with OCH₃, OH or OC₂H₅, in particular with OCH₃.

5. Spirocyclic cyclohexane derivatives according to claim 1, wherein the radical R⁵ denotes H, CH₃, COOH, COOCH₃, CH₂O-phenyl, wherein the phenyl radical can be substituted with F, Cl, Br, I, CN, CH₃, C₂H₅, NH₂, NO₂, SH, CF₃, OH, OCH₃, OC₂H₅ or N(CH₃)₂, or CH₂OH.

6. Spirocyclic cyclohexane derivatives according to claim 1, wherein R⁶ can denote H; methyl, ethyl, CF₃, benzyl or phenyl, wherein the benzyl or phenyl radical can be substituted with F, Cl, Br, I, CN, CH₃, C₂H₅, NH₂, NO₂, SH, CF₃, OH, OCH₃, OC₂H₅ or N(CH₃)₂.

7. Spirocyclic cyclohexane derivatives according to claim 1, wherein R⁷, R⁸, R⁹ and R¹⁰ independently of one another denote H; C₁₋₅ alkyl, branched or unbranched, unsubstituted or mono- or polysubstituted; F, Cl, Br, I, CF₃, OH, OCH₃, NH₂, COOH, COOCH₃, NHCH₃, thienyl, pyrimidinyl, pyridyl, N(CH₃)₂ or NO₂.

8. Spirocyclic cyclohexane derivatives according to one of claims 1 to 7, wherein
X denotes O.

9. Spirocyclic cyclohexane derivatives according to one of claims 1 to 7, wherein X denotes NR¹⁷.

10. Spirocyclic cyclohexane derivatives according to claim 1, from the group comprising
N-{6'-Fluoro-4',9'-dihydro-4-phenylspiro[cyclohexane-1,1'(3'H)-pyrano[3,4-b]indol]-4-yl}-azetidine, 2-hydroxy-1,2,3-propanetricarboxylate (2:1)
4-(Azetidin-1-yl)-4-(3-fluorophenyl)-2',3',4',9'-tetrahydrospiro[cyclohexane-1,1'-pyrido[3,4-b]indole] (polar diastereomer)
4-(Azetidin-1-yl)-4-(3-fluorophenyl)-2',3',4',9'-tetrahydrospiro[cyclohexane-1,1'-pyrido[3,4-b]indole] (non-polar diastereomer)
4-(Azetidin-1-yl)-4-(3-fluorophenyl)-4',9'-dihydro-3'H-spiro[cyclohexane-1,1'-pyrano[3,4-b]indole] (one of two possible diastereomers)
1-(4-(Azetidin-1-yl)-4-(3-fluorophenyl)-3',4'-dihydrospiro[cyclohexane-1,1'-pyrido[3,4-b]indol]-2'(9'H)-yl)-3-phenylprop-2-en-1-one (polar diastereomer)
4-(Azetidin-1-yl)-6'-fluoro-4-(thiophen-2-yl)-4',9'-dihydro-3'H-spiro[cyclohexane-1,1'-pyrano[3,4-b]indole] 2-hydroxypropane-1,2,3-tricarboxylate (1:1) (non-polar diastereomer)
optionally also as a mixture.

11. Process for the preparation of spirocyclic cyclohexane derivatives according to claim 1, **characterised in that** a reactant having the general formula E 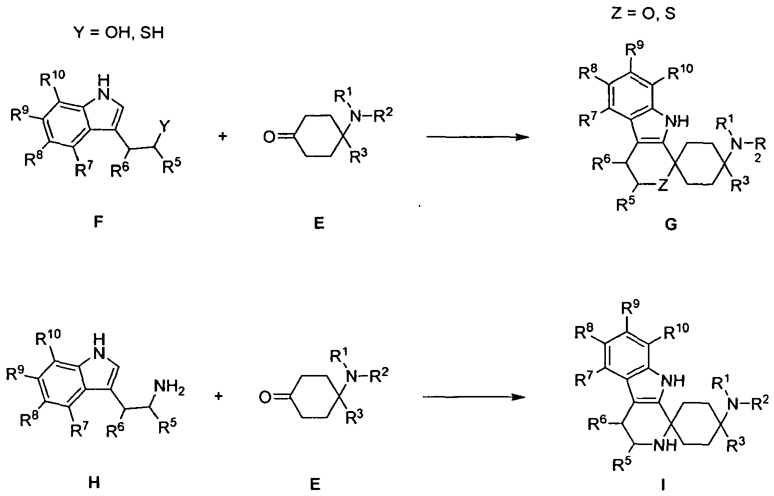 is reacted with addition of acid or the trimethylsilyl esters thereof, for example trifluoromethanesulfonic acid trimethylsilyl ester, trifluoromethanesulfonic acid, acetic acid, phosphoric acid, methanesulfonic acid or trifluoroacetic acid, in a suitable solvent, for example dichloroethane, dichloromethane, chloroform, acetonitrile, diethyl ether or nitromethane, with a reactant having the general formula F or H, wherein the radicals R¹ to R³ and R⁵ to R¹⁰ have the meanings given in claim 1.

12. Process for the preparation of spirocyclic cyclohexane derivatives according to claim 1, wherein X denotes NR¹⁷ and R¹⁷ denotes COR¹² or SO₂R¹², **characterised in that** a spirocyclic cyclohexane derivative in which X denotes NH is reacted with an anhydride or an acid chloride with addition of a base, for example triethylamine, preferably under microwave radiation.

13. Process for the preparation of spirocyclic cyclohexane derivatives according to claim 1, wherein X denotes SO or SO₂, **characterised in that** a spirocyclic cyclohexane derivative in which X denotes S is oxidised with the aid of an oxidising agent, for example H₂O₂.

14. Medicament containing at least one spirocyclic cyclohexane derivative according to one of claims 1 to 10, optionally in the form of its racemate, the pure stereoisomers, in particular enantiomers and diastereomers, in any mixing ratio; in the form of its acids or its bases or in the form of its salts, in particular the physiologically compatible salts or salts of physiologically compatible acids or cations; or in the form of its solvates, in particular the hydrates, and optionally containing suitable additives and/or auxiliary substances and/or optionally further active ingredients.

15. Use of a spirocyclic cyclohexane derivative according to one of claims 1 to 10, optionally in the form of its racemate, the pure stereoisomers, in particular enantiomers and diastereomers, in any mixing ratio; in the form of its acids or its bases or in the form of its salts, in particular the physiologically compatible salts or salts of physiologically compatible acids or cations; or in the form of its solvates, in particular the hydrates, for the preparation of a medicament for the treatment of pain, in particular acute, neuropathic or chronic pain.

16. Use of a spirocyclic cyclohexane derivative according to one of claims 1 to 10 to prepare a medicament for the treatment of anxiety conditions, stress and stress-related syndromes, depression, epilepsy, Alzheimer's disease, senile dementia, general cognitive dysfunctions, learning and memory disorders (as a nootropic), withdrawal symptoms, alcohol and/or drug and/or prescription drug abuse and/or dependency, sexual dysfunctions, cardiovascular diseases, hypotension, hypertension, tinnitus, pruritus, migraine, hearing impairment, gastrointestinal motility disorders, food intake disorders, anorexia, obesity, locomotive disorders, diarrhoea, cachexia, urinary incontinence, or as a muscle relaxant, anticonvulsant or anaesthetic, or for coadministration in treatment with an opioid analgesic or with an anaesthetic, for diuresis or antinatriuresis, anxiolysis, for the modulation of motor activity, for the modulation of neurotransmitter release and treatment of associated neurodegenerative diseases, for the treatment of withdrawal symptoms and/or for the reduction of the addiction potential of opioids.

## Revendications

1. Dérivés spirocycliques de cyclohexane de formule
générale I, 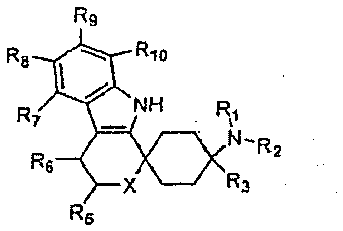 dans laquelle,
R¹ et R² forment ensemble un cycle et représentent un groupe -CH₂CH₂CH₂- ;
R³ représente un groupe alkyle en C₁ à C₅, dans chaque cas saturé ou non saturé, ramifié ou non ramifié, substitué une ou plusieurs fois ou non substitué ; un groupe cycloalkyle en C₃ à C₈, dans chaque cas saturé ou non saturé, substitué une ou plusieurs fois ou non substitué ; un groupe aryle ou un hétéroaryle, dans chaque cas non substitué ou substitué une ou plusieurs fois ; un groupe, lié par l'intermédiaire d'un groupe alkyle en C₁ à C₃, aryle ou cycloalkyle en C₃ à C₈, dans chaque cas non substitué ou substitué une ou plusieurs fois ;
R⁵ représente =O ; H ; un groupe alkyle en C₁ à C₅, saturé ou non saturé, ramifié ou non ramifié, non substitué ou substitué une ou plusieurs fois ; COOR¹³, CONR¹³, OR¹³ ; un groupe cycloalkyle en C₃ à C₈, saturé ou non saturé, non substitué ou substitué une ou plusieurs fois ; un groupe aryle ou hétéroaryle, non substitué ou substitué une ou plusieurs fois ; ou un groupe, lié par l'intermédiaire d'un groupe alkyle en C₁ à C₃, aryle, cycloalkyle en C₃ à C₈ ou hétéroaryle, non substitué ou substitué une ou plusieurs fois ;
R⁶ représente H ; F, Cl, NO₂, CF₃, OR¹³, SR¹³, SO₂R¹³, SO₂OR¹³, CN, COOR¹³, NR¹⁴R¹⁵ ; un groupe alkyle en C₁ à C₅, saturé ou non saturé, ramifié ou non ramifié, non substitué ou substitué une ou plusieurs fois ; un groupe cycloalkyle en C₃ à C₈, saturé ou non saturé, non substitué ou substitué une ou plusieurs fois ; un groupe aryle ou hétéroaryle, non substitué ou substitué une ou plusieurs fois ; ou un groupe, lié par l'intermédiaire d'un groupe alkyle en C₁ à C₃, aryle, cycloalkyle en C₃ à C₈ ou hétéroaryle, non substitué ou substitué une ou plusieurs fois ;
ou R⁵ et R⁶ forment ensemble un groupe (CH₂) n, dans lequel n est égal à 2, 3, 4, 5 ou 6, des atomes individuels d'hydrogène pouvant aussi être remplacés par F, Cl, Br, I, NO₂, CF₃, OR¹³, CN ou un groupe alkyle en C₁ à C₅ ;
R⁷, R⁸, R⁹ et R¹⁰ représentent, indépendamment les uns des autres, H, F, Cl, Br, I, NO₂, CF₃, OR¹³, SR¹³, SO₂R¹³, SO₂OR¹³, NHC (=O) NR¹⁴R¹⁵, SO₂NR¹⁴R¹⁵, CN, COOR¹³, NR¹⁴R¹⁵ ; un groupe alkyle en C₁ à C₅, cycloalkyle en C₃ à C₈, non substitué ou substitué une ou plusieurs fois ; un groupe aryle ou hétéroaryle, non substitué ou substitué une ou plusieurs fois ; ou un groupe, lié par l'intermédiaire d'un groupe alkyle en C₁ à C₃, aryle, cycloalkyle en C₃ à C₈ ou hétéroaryle, non substitué ou substitué une ou plusieurs fois ;
R¹³ représente H ; un groupe alkyle en C₁ à C₅, dans chaque cas saturé ou non saturé, ramifié ou non ramifié, non substitué ou substitué une ou plusieurs fois ; un groupe cycloalkyle en C₃ à C₈, dans chaque cas saturé ou non saturé, non substitué ou substitué une ou plusieurs fois ; un groupe aryle ou hétéroaryle, non substitué ou substitué une ou plusieurs fois ; ou un groupe, lié par l'intermédiaire d'un groupe alkyle en C₁ à C₃, aryle, cycloalkyle en C₃ à C₈ ou hétéroaryle, non substitué ou substitué une ou plusieurs fois ;
R¹⁴ et R¹⁵ représentent, indépendamment l'un de l'autre, H ; un groupe alkyle en C₁ à C₅, dans chaque cas saturé ou non saturé, ramifié ou non ramifié, non substitué ou substitué une ou plusieurs fois ; ou un groupe cycloalkyle en C₃ à C₈, dans chaque cas saturé ou non saturé, non substitué ou substitué une ou plusieurs fois ; un groupe aryle ou hétéroaryle, non substitué ou substitué une ou plusieurs fois ; ou un groupe, lié par l'intermédiaire d'un groupe alkyle en C₁ à C₃, aryle, cycloalkyle en C₃ à C₈ ou hétéroaryle, non substitué ou substitué une ou plusieurs fois ;
ou R¹⁴ et R¹⁵ forment ensemble un groupe CH₂CH₂OCH₂CH₂, CH₂CH₂NR¹⁶CH₂CH₂ ou (CH₂)₃₋₆,
R¹⁶ représente H ; un groupe alkyle en C₁ à C₅, saturé ou non saturé, ramifié ou non ramifié, non substitué ou substitué une ou plusieurs fois ;
X représente O, S, SO, SO₂ ou NR¹⁷ ;
R¹⁷ représente H ; un groupe alkyle en C₁ à C₅, saturé ou non saturé, ramifié ou non ramifié ; COR¹² ou SO₂R¹²,
R¹² représentant H ; un groupe alkyle en C₁ à C₅, saturé ou non saturé, ramifié ou non ramifié, substitué une ou plusieurs fois ou non substitué ; un groupe cycloalkyle en C₃ à C₈, dans chaque cas saturé ou non saturé, substitué une ou plusieurs fois ou non substitué ; un groupe aryle ou hétéroaryle, dans chaque cas substitué une ou plusieurs fois ou non substitué ; ou un groupe, lié par l'intermédiaire d'un groupe alkyle en C₁ à C₃, aryle, cycloalkyle en C₃ à C₈ ou hétéroaryle, dans chaque cas non substitué ou substitué une ou plusieurs fois ; OR¹³ ; NR¹⁴R¹⁵ ;
sous la forme des racémates ; des énantiomères, des diastéréoisomères, des mélanges des énantiomères ou des diastéréoisomères, ou d'un énantiomère ou diastéréoisomère individuel ; des bases et/ou des sels d'acides ou de cations physiologiquement acceptables.

2. Dérivés spirocycliques de cyclohexane selon la revendication 1, dans lesquels
« alkyle substitué » ou « cycloalkyle substitué » représente un groupe alkyle ou cycloalkyle substitué par F, Cl, Br, I, CN, CH₃, C₂H₅, NH₂, NO₂, SH, CF₃, OH, OCH₃, OC₂H₅ ou N(CH₃)₂ et dans lesquels
« aryle substitué » ou « hétéroaryle substitué » représente un groupe alkyle ou hétéroaryle substitué par F, Cl, Br, I, CN, CH₃, C₂H₅, NH₂, NO₂, SH, CF₃, OH, OCH₃, OC₂H₅ ou N(CH₃)₂.

3. Dérivés spirocycliques de cyclohexane selon la revendication 1, dans lesquels
R³ représente un groupe phényle, benzyle ou phénéthyle, dans chaque cas non substitué ou substitué une ou plusieurs fois au niveau du cycle ; un groupe alkyle en C₁ à C₅, non substitué ou substitué une ou plusieurs fois ; un groupe cycloalkyle en C₄ à C₈, non substitué ou substitué une ou plusieurs fois ; un groupe pyridyle, thiényle, thiazolyle, imidazolyle, 1,2,4-triazolyle ou benzimidazolyle, non substitué ou substitué une ou plusieurs fois.

4. Dérivés spirocycliques de cyclohexane selon la revendication 1, dans lesquels
R³ représente un groupe phényle, non substitué ou substitué une ou plusieurs fois par F, Cl, CN, CH₃ ; un groupe thiényle ; un groupe éthyle, n-propyle ou n-butyle, non substitué ou substitué une ou plusieurs fois par OCH₃, OH ou OC₂H₅, en particulier par OCH₃.

5. Dérivés spirocycliques de cyclohexane selon la revendication 1, dans lesquels
le reste R⁵ représente H, CH₃, COOH, COOCH₃, CH₂O-phényle, le reste phényle pouvant être substitué par F, Cl, Br, I, CN, CH₃, C₂H₅, NH₂, NO₂, SH, CF₃, OH, OCH₃, OC₂H₅ ou N(CH₃)₂, ou CH₂OH .

6. Dérivés spirocycliques de cyclohexane selon la revendication 1, dans lesquels
R⁶ peut représenter H ; un groupe méthyle, éthyle, CF₃, benzyle ou phényle, le groupe benzyle ou phényle pouvant être substitué par F, Cl, Br, I, CN, CH₃, C₂H₅, NH₂, NO₂, SH, CF₃, OH, OCH₃, OC₂H₅ ou N(CH₃)₂.

7. Dérivés spirocycliques de cyclohexane selon la revendication 1, dans lesquels
R⁷, R⁸, R⁹ et R¹⁰ représentent, indépendamment les uns des autres, H ; un groupe alkyle en C₁ à C₅, ramifié ou non ramifié, non substitué ou substitué une ou plusieurs fois ; F, Cl, Br, I, CF₃ , OH , OCH₃ , NH₂, COOH, COOCH₃ , NHCH₃, thiényle, pyrimidinyle, pyridyle, N(CH₃)₂ ou NO₂.

8. Dérivés spirocycliques de cyclohexane selon l'une des revendications 1 à 7, où X représente O.

9. Dérivés spirocycliques de cyclohexane selon l'une des revendications 1 à 7, où X représente NR¹⁷.

10. Dérivés spirocycliques de cyclohexane selon la revendication 1, choisis dans le groupe des composés suivantes :
N-{6'-fluoro-4',9'-dihydro-4-phényl-spiro[cyclohexane-1,1'(3'H)-pyranno[3,4-b]indole]-4-yl}-azétidine, 2-hydroxy-1,2,3-tricarboxylate de propane (2/1)
4-(azétidine-1-yl)-4-(3-fluorophényl)-2',3',4',9'-tétrahydrospiro[cyclohexane-1,1'-pyrido[3,4-b]indole] (diastéréoisomère le plus polaire)
4-(azétidine-1-yl)-4-(3-fluorophényl)-2',3',4',9'-tétrahydrospiro[cyclohexane-1,1'-pyrido[3,4-b]indole] (diastéréoisomère le moins polaire)
4-(azétidine-1-yl)-4-(3-fluorophényl)-4',9'-dihydro-3'H-spiro[cyclohexane-1,1'-pyranno[3,4-b]indole] (un des 2 diastéréoisomères possibles)
1-(4-(azétidine-1-yl)-4-(3-fluorophényl)-3',4'-dihydrospiro[cyclohexane-1,1'-pyranno[3,4-b]indole]-2'(9'H)-yl)-3-phénylprop-2-èn-1-one (diastéréoisomère le plus polaire)
4-(azétidine-1-yl)-6'-fluoro-4-(thiophène-2-yl)-4',9'-dihydro-3'H-spiro[cyclohexane-1,1'-pyrido[3,4-b]indole], 2-hydroxypropane-1,2,3-tricarboxylate (1/1) (diastéréoisomère le moins polaire)
le cas échéant également sous forme de mélange.

11. Procédé de fabrication de dérivés spirocycliques de cyclohexane selon la revendication 1, **caractérisé en ce qu'**un composé de départ de formule générale E 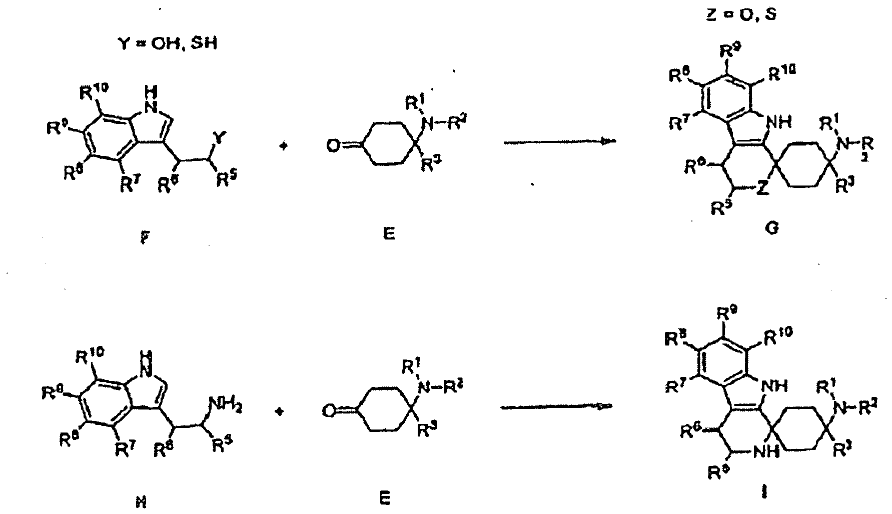 avec addition d'un acide ou de son ester de triméthylsilyle, par exemple l'ester triméthylsilylique d'acide trifluorométhanesulfonique, l'acide trifluorométhanesulfonique, l'acide acétique, l'acide phosphorique, l'acide méthanesulfonique ou l'acide trifluoracétique, dans un solvant approprié, par exemple le dichloréthane, le dichlorométhane, le chloroforme, l'acétonitrile, l'éther de diéthyle ou le nitrométhane, est amené à réagir avec un composé de départ de formule générale F ou H, les groupes R¹ à R³ et R⁵ à R¹⁰ ayant les définitions indiquées dans la revendication 1.

12. Procédé de production de dérivés spirocycliques de cyclohexane selon la revendication 1, dans lesquels X représente NR¹⁷ et R¹⁷ représente un groupe COR¹² ou SO₂R¹², **caractérisé en ce qu'**un dérivé spirocyclique de cyclohexane, dans lequel X représente NH, est amené à réagir avec un anhydride ou un chlorure d'acide, avec addition d'une base, par exemple la triéthylamine, de préférence sous irradiation par micro-ondes.

13. Procédé de production de dérivés spirocycliques de cyclohexane selon la revendication 1, dans lesquels X représente SO ou SO₂, **caractérisé en ce qu'**un dérivé spirocyclique de cyclohexane, dans lequel X représente S, est oxydé à l'aide d'un agent oxydant, par exemple H₂O₂.

14. Médicament contenant au moins un dérivé spirocyclique de cyclohexane selon l'une des revendications 1 à 10, le cas échéant sous la forme de son racémate, des stéréoisomères purs, en particulier des énantiomères et des diastéréoisomères, dans un rapport de mélange quelconque ;
sous la forme de ses acides ou de ses bases ou sous la forme de ses sels, en particulier des sels physiologiquement acceptables ou de sels d'acides ou de cations physiologiquement acceptables ; ou sous la forme de ses produits de solvatation, en particulier des hydrates,
contenant le cas échéant des additifs et/ou des substances appropriés et/ou le cas échéant d'autres substances actives.

15. Utilisation d'un dérivé spirocyclique de cyclohexane selon l'une des revendications 1 à 10, le cas échéant sous la forme de son racémate, des stéréoisomères purs, en particulier des énantiomères et des diastéréoisomères, dans un rapport de mélange quelconque ;
sous la forme de ses acides ou de ses bases ou sous la forme de ses sels, en particulier des sels physiologiquement acceptables ou de sels d'acides ou de cations physiologiquement acceptables ; ou sous la forme de ses produits de solvatation, en particulier des hydrates ; pour la préparation d'un médicament destiné au traitement de la douleur, en particulier d'une douleur aiguë, neuropathique ou chronique.

16. Utilisation d'un dérivé spirocyclique de cyclohexane selon l'une des revendications 1 à 10, pour la préparation d'un médicament destiné au traitement d'états d'anxiété, de stress et de syndromes liés au stress, de dépressions, de l'épilepsie, de la maladie d'Alzheimer, de la démence sénile, de dysfonctionnements cognitifs généraux, de troubles de l'apprentissage et de la mémoire (comme nootrope), de phénomènes inflammatoires, de l'abus d'alcool et/ou de drogues et/ou de médicaments et/ou de la dépendance à l'égard de l'alcool et/ou des drogues et/ou des médicaments, de dysfonctionnements sexuels, de maladies cardiovasculaires, de l'hypotension, de l'hypertension, des acouphènes, du prurit, de la migraine, de la surdité, de l'insuffisance de motilité intestinale, de troubles de l'absorption de nourriture, de l'anorexie, de l'obésité, de troubles locomoteurs, de la diarrhée, de la cachexie, de l'incontinence urinaire ou comme myorelaxant, anticonvulsif ou anesthésique ou en vue d'une coadministration dans un traitement avec un analgésique opioïde ou avec un anesthésique, pour la diurèse ou l'antinatriurèse, l'anxiolyse, la modulation de l'activité motrice, la modulation de l'émission de neurotransmetteurs et le traitement de maladies neurodégénératives qui y sont liées, pour le traitement de phénomènes inflammatoires et/ou pour la réduction du potentiel de dépendance aux opioïdes.
